# EUROPEAN PATENT APPLICATION

(11) **EP 0 982 388 A1**
(43) Date of publication of application: **01.03.2000**
(21) Application number: 98115938.7
(22) Date of filing: 24.08.1998
(51) Int. Cl.: C09K 19/04, C09K 19/30, C09K 19/34, C09K 19/32, C07C 23/18

(54) **Liquid crystal compound having negative dielectric anisotropy, liquid crystal composition containing said liquid compound and liquid crystal display device using said composition**

(71) Applicant: Chisso Corporation, Osaka-shi Osaka 530-0055 (JP)
(72) Inventor: Miyazawa, Kazutoshi, Ichihara-shi, Chiba-ken (JP); Yano, Hitoshi, Ichihara-shi, Chiba-ken (JP)
(74) Representative: Müller-Boré & Partner Patentanwälte

(57) **Abstract**

A liquid crystal compound which has a 2,3-difluorocyclohexa-1,3-diene-1,4-diyl group in the general structure of the compound. The liquid crystal compound has a negative dielectric anisotropy value whose absolute value is large, a low viscosity, a controlled optical anisotropy value, a high specific resistance and a high voltage-holding ratio and is stable against heat and irradiation with ultraviolet rays. Accordingly, the compound is effective for preparing a liquid crystal composition having negative dielectric anisotropy and a liquid crystal display device.

## Description

The present invention mainly relates to a novel liquid crystal compound having characteristic properties favorable for a liquid crystal composition used for liquid crystal display devices of various display modes such as vertically aligned mode, IPS (in-plane switching) mode, TFT (thin film transistor) mode, TN (twisted nematic) mode or STN (super twisted nematic) mode, in particular, vertically aligned mode and IPS mode as well as a liquid crystal composition having excellent characteristic properties and comprising the liquid crystal compound and a liquid crystal display device produced using the liquid crystal composition. In the present invention, the term "liquid crystal compound" is a general term for compounds capable of exhibiting liquid crystal phases and compounds which do not exhibit any liquid crystal phase, but are useful as ingredients for liquid crystal compositions.

Up to this time, there have been proposed various liquid crystal display modes. As an example thereof, there can be listed the following display mode (see "Up-To-Date Liquid Crystal Technique", edited by Kogyochosa Kai, 1983). Examples of modes which make use of liquid crystal compositions having positive dielectric anisotropy are TN modes, STN modes, or TN based AM (active matrix) modes (such as TFT or MIM (metal-insulating film-metal) modes). Moreover, as modes which make use of liquid crystal compositions having negative dielectric anisotropy, there may be listed, for instance, ECB (Electric field-control birefringent effect) modes (such as HAN (hybrid molecular arrangement) mode or DAP (vertical molecular arrangement) mode), DS (dynamic scattering) mode, GH (guest-host) mode or PC (phase transition) mode.

Among these modes, the mainstreams thereof which have presently been used are those which make use of liquid crystal compositions having positive dielectric anisotropy. Contrary to this, there has been a delay in the practical application of the modes utilizing liquid crystal compositions having negative dielectric anisotropy. In connection with this, the development of liquid crystal compositions having negative dielectric anisotropy and compounds per se used in the compositions have also been insufficient as compared with the development of such compositions having positive dielectric anisotropy and compounds used in these compositions.

Under these circumstances, there have recently been reported wide variety of attempts, in particular, to eliminate one of disadvantages of the liquid crystal display techniques, i.e., the narrowness of the viewing angle. An example thereof is IPS mode (see, for instance, R. Kiefer et al., JAPAN DISPLAY '92, 547 (1992); M. Oh-e et al., ASIA DISPLAY '95, 577 (1995); TOKUHYO Hei 5-505247; Japanese Un-Examined Patent Publication (hereinafter referred to as "J.P. KOKAI") No. Hei 7-12864 7). One of the characteristic properties of IPS mode is that a comb-like electrode is arranged only on one substrate in the liquid crystal panel, while electrodes are formed on the both upper and lower substrates in the conventional liquid crystal panel. Another characteristic property of the mode is that a liquid crystal composition can be used irrespective of the dielectric anisotropy of the composition (positive or negative).

As another example of the attempts to extend the angle of vision, there may be listed modes wherein the viewing angle is improved by making use of the vertical alignment of liquid crystal molecules (see, for instance, J.P. KOKAI No. Hei 2-176625). One of the characteristic properties of this mode is to use a liquid crystal composition having negative dielectric anisotropy.

With these points as background, it has intensively been required for the development of a liquid crystal compound having negative dielectric anisotropy and a liquid crystal composition.

Incidentally, in all of the display modes, the liquid crystal compositions used therein should have not only an appropriate dielectric anisotropy value, but also the best-controlled other characteristic properties such as values of optical anisotropy (Δn), elastic constant ratio: K₃₃/K₁₁ (K₃₃: bend elastic constant; K₁₁: spray elastic constant) and further they must satisfy such requirements that the temperature of the liquid crystal phase falls within an appropriate range and that they should have a low viscosity even at a low temperature.

None of conventionally known liquid crystal compounds satisfy all of these requirements when they are used alone. For this reason, a composition which can be used as a liquid crystal material has usually been prepared by mixing several kinds to twenty or so kinds of compounds capable of exhibiting liquid crystal phases and optionally several kinds of compounds incapable of exhibiting any liquid crystal phase. Therefore, each liquid crystal compound should also satisfy such requirements that it has good miscibility with other liquid crystal compounds and that it also has good miscibility therewith in a low temperature range since the composition is also used in low temperature environments.

As has been described above, however, it has been the mainstream to use liquid crystal compositions comprising liquid crystal compounds having positive dielectric anisotropy in the conventional display modes and the development of liquid crystal compositions and compounds having negative dielectric anisotropy have still been insufficient. Therefore, the conventional liquid crystal compounds and compositions have not been able to completely cope with such diversified modes and related various characteristics. For instance, they suffer from the following various problems:
. They have negative dielectric anisotropy, but the absolute value thereof is small;
. They do not permit the reduction of the driving voltage of the display device because of their large elastic constants;
. They cannot be used in a large amount because of poor miscibility with other ingredients for the liquid crystal compositions and display devices using them;
. It is difficult to freely establish the optical anisotropy of these compositions or compounds;
. They have high viscosities; and
. They are physically and chemically less stable.

There has already been reported liquid crystal compounds each having a cyclohexa-1,3-dien-1,4-diyl group (M.J.S. Dewar et al., J. Am. Chem. Soc., 1975,97, p.6662). However, the compounds disclosed in this article do not have any fluorine atom on the cyclohexa-1,3-dien-1,4-diyl group. Moreover, these compounds do not necessarily show satisfactory physical properties required for use in liquid crystal compositions such as appropriate dielectric anisotropy and optical anisotropy values, low elastic constants, good miscibility, low viscosities and high chemical and physical stability and, in particular, they do not have negative dielectric anisotropy values whose absolute values are large. For this reason, there has been desired for further improvement of these compounds and compositions.

In addition, there has already reported, prior to the application of this invention, compound each having a cyclohexa-1,3-diene-1,4-diyl group and whose hydrogen atom (or atoms) on the ring is replaced with a fluorine atom (or atoms) (see, for instance, M. Zupan et al., J. Org. Chem., 1978, 43(11), p. 2297). However, the fluorine atoms of the compounds disclosed in this article are not position-selectively introduced thereinto, they do not have an ability of exhibiting a liquid crystal phase and there is not any disclosure in the article to the effect that they can be used for exhibiting a liquid crystal phase. More specifically, there are not described therein, for instance, appropriate dielectric anisotropy and optical anisotropy values, low elastic constants, good miscibility, low viscosities and high chemical and physical stability of these compounds.

In particular, there is not disclosed, in these reports, such an idea that the compounds each having a cyclohexa-1,3-diene-1,4-diyl group are converted into liquid crystal compounds having negative dielectric anisotropy values. More specifically, there has yet not been proposed any idea that substituents are selectively introduced into the 2- and/or 3-positions of cyclohexa-1,3-diene-1,4-diyl group.

As has been described above, the compounds of the present invention cannot easily be hit upon on the basis of the teachings or knowledges of the prior art.

Accordingly, an object of the present invention is to provide a novel liquid crystal compound which can be not only used in various display modes utilizing compounds or compositions having negative dielectric anisotropy, such as vertically aligned mode as disclosed in J.P. KOKAI No. Hei 2-176625, IPS mode, ECB mode (such as HAN mode or DAP mode), DS mode, GH mode or PC mode, but also used for controlling various characteristics of liquid crystal compositions for various display modes utilizing compounds or compositions having positive dielectric anisotropy, such as TN, STN, or TN based AM (TFT or MIM) mode, and which can solve the foregoing problems, as well as a liquid crystal composition containing the compound and a liquid crystal display device produced using the composition.

The inventors of this invention have conducted various studies to accomplish the foregoing objects, as a result, have found that the compounds as specified in the following first aspect and a related embodiment of the present invention not only have negative dielectric anisotropy whose absolute values are large, but also have appropriate optical anisotropy, small elastic constants, good miscibility with other substances, low viscosities and high physicochemical stability, that the use of the foregoing compounds permits the formation of liquid crystal compositions whose elastic constant is small, whose dielectric and optical anisotropy values can appropriately be established, which have low viscosities and excellent physicochemical stability and, in particular, which have negative dielectric anisotropy whose absolute values are large and that an excellent liquid crystal display device can be obtained by the use of the composition and thus have completed the present invention.

According to a first aspect of the present invention, there is provided a liquid crystal compound having a 2,3-difluorocyclohexa-1,3-diene-1,4-diyl group in the general structure of the compound.

The compound is preferably those represented by the following general formula (1):

R¹-(A¹-X¹)ₐ₁-(A²-X²)ₐ₂-(A³-X³)ₐ₃-(A⁴-X⁴)ₐ₄-(A⁵)ₐ₅-Y¹ (1)

wherein R¹ and Y¹ each independently represents an alkyl group having 1 to 20 carbon atoms, a hydrogen atom, a halogen atom, a cyano group, a cyanate residue, an isocyano group or an isothiocyanate residue, provided that at least one of the methylene groups present in the alkyl group, which are not adjacent to one another, may be substituted with an oxygen, sulfur or nitrogen atom, -C ≡C-, a silylene group wherein at least one hydrogen atom may be substituted with an alkyl group having 1 to 10 carbon atoms, or a vinylene group, or that at least one hydrogen atom of the alkyl group may be substituted with a fluorine or chlorine atom or that these alkyl groups may be optically active ones; X¹, X², X³ and X⁴ each independently represents a single bond, -(CH₂)₂-, -CH=CH-, -C≡C-, -COO-, -OCO-, -CH₂O-, -OCH₂-, -(CH₂)₄-, -(CH₂)₃O-, -O(CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, -CF₂CH₂-, -(CF₂)₂-, -(CF₂)₄-, -(CH₂)₂COO-, -OCO(CH₂)₂-, -CH=CHCOO-, -OCOCH=CH-, -CH=CHC≡C- or -C≡CCH=CH-; the rings A¹, A², A³, A⁴ and A⁵ each independently represents a 2,3-difluorocyclohexa-1, 3-diene-1,4-diyl, bicyclo[1.1.1]pentane-1,3-diyl, trans-cyclohexane-1,4-diyl, cyclohexa-1-ene-1,4-diyl or 1,4-phenylene group, provided that the carbon atoms constituting these rings may be substituted with a nitrogen, oxygen or sulfur atom, that each hydrogen atom present on the rings may be substituted with a halogen atom or a cyano group and that at least one of these rings A¹, A², A³, A⁴ and A⁵ is 2,3-difluorocyclohexa-1,3-diene-1,4-diyl; a¹, a², a³, a⁴ and a⁵ each independently represents 0 or 1, provided that they satisfy the relation: a¹+a²+a³+a⁴+a⁵ ≧1; and each atom constituting the compound may be substituted with its isotope.

According to a second aspect of the present invention, there is provided a liquid crystal composition which comprises at least two components including at least one liquid crystal compound defined above.

According to a third aspect of the present invention, there is provided a liquid crystal composition which comprises at least one liquid crystal compound defined above as a first component and at least one compound selected from the group consisting of those represented by the following general formulas (2), (3) and (4) as a second component: 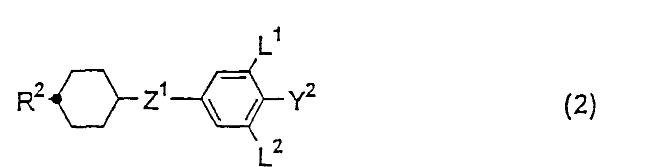 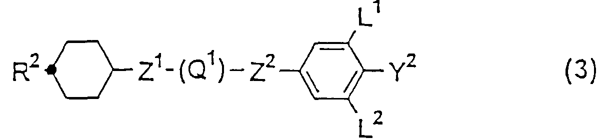 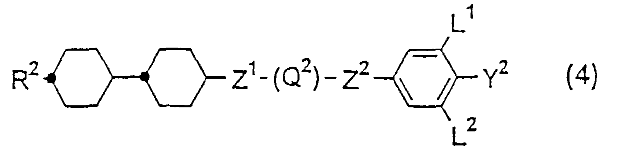 wherein R² represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y² represents a fluorine or chlorine atom, -OCF₃, -OCF₂H, -CF₃, -CF₂H, -CFH₂, -OCF₂CF₂H or -OCF₂CFHCF₃; L¹ and L² each independently represents a hydrogen or fluorine atom; Z¹ and Z² each independently represents - (CH₂)₂-, -(CH₂)₄-, -COO-, -CF₂O-, -OCF₂-, -CH=CH- or a single bond; the ring Q¹ represents a trans-cyclohexane-1,4-diyl or 1,3-dioxane-2,5-diyl group, or a 1,4-phenylene group whose hydrogen atom may be substituted with a fluorine atom; the ring Q² represents a trans-cyclohexane-1,4-diyl group, or a 1,4-phenylene group whose hydrogen atom may be substituted with a fluorine atom; and each atom constituting the compound may be substituted with its isotope.

According to a fourth aspect of the present invention, there is provided a liquid crystal composition which comprises at least one liquid crystal compound defined above as a first component and at least one compound selected from the group consisting of those represented by the following general formulas (5) and (6) as a second component: 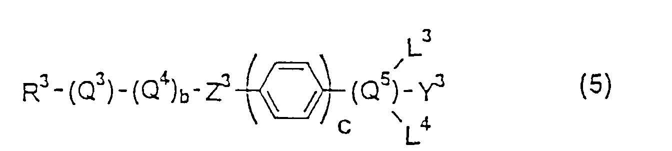 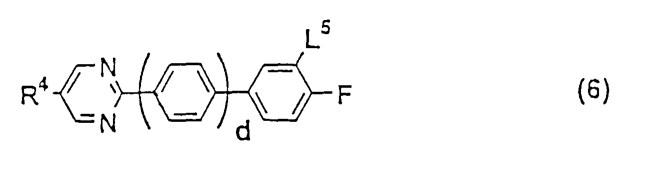 wherein R³ and R⁴ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y³ represents a cyano group or -C ≡C-CN; the ring Q³ represents a trans-cyclohexane-1,4-diyl, 1,4-phenylene, 1,3-dioxane-2,5-diyl or pyrimidine-2,5-diyl group; the ring Q⁴ represents a trans-cyclohexane-1,4-diyl or pyrimidine-2,5-diyl group, or a 1,4-phenylene group whose hydrogen atom may be substituted with a fluorine ato m; the ring Q⁵ represents a trans-cyclohexane-1,4-diyl or 1,4-phenylene group; Z³ represents -(CH₂)₂-, -COO- or a single bond; L³, L⁴ and L⁵ each independently represents a hydrogen or fluorine atom; b, c and d each independently represents 0 or 1; and each atom constituting the compound may be substituted with its isotope.

According to a fifth aspect of the present invention, there is provided a liquid crystal composition which comprises at least one liquid crystal compound defined above as a first component; at least one compound selected from the group consisting of those represented by the foregoing general formulas (2), (3) and (4) as a second component; and at least one compound selected from the group consisting of those represented by the following general formulas (7), (8) and (9) as a third component:

R⁵-(Q⁶)-Z⁴-(Q⁷)-Z⁵-R⁶ (7)

R⁵-(Q⁶)-Z⁴-(Q⁷)-Z⁵-(Q⁸)-R⁶ (8)

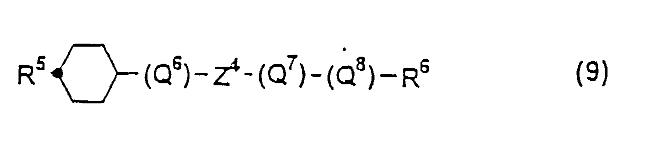

wherein R⁵ and R⁶ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁶, Q⁷ and Q⁸ each independently represents a trans-cyclohexane-1,4-diyl or pyrimidine-2,5-diyl group, or a 1,4-phenylene group whose hydrogen atom may be substituted with a fluorine ato m; Z⁴ and Z⁵ each independently represents -C≡C-, -COO-, -(CH₂)₂-, -CH=CH- or a single bond; and each atom constituting the compound may be substituted with its isotope.

According to a sixth aspect of the present invention, there is provided a liquid crystal composition which comprises at least one liquid crystal compound defined above as a first component and at least one compound selected from the group consisting of those represented by the following general formulas (10), (11) and (12) as a second component: 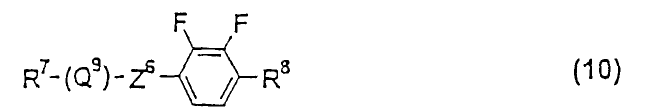 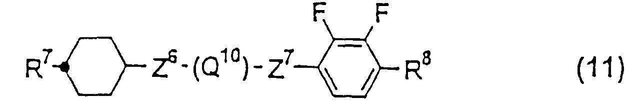 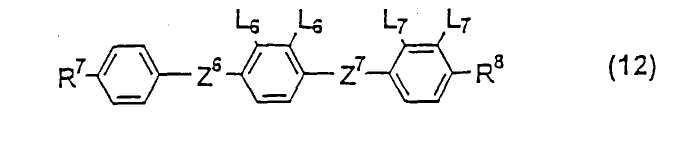 wherein R⁷ and R⁸ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁹ and Q¹⁰ each independently represents a trans-cyclohexane-1,4-diyl or 1,4-phenylene group; L⁶ and L⁷ each independently represents a hydrogen or fluorine atom, provided that L⁶ and L⁷ do not simultaneously represent a hydrogen atom; Z⁶ and Z⁷ each independently represents -(CH₂)₂-, -COO- or a single bond; and each atom constituting the compound may be substituted with its isotope.

According to a seventh aspect of the present invention, there is provided a liquid crystal composition which comprises at least one liquid crystal compound defined above as a first component; at least one compound selected from the group consisting of those represented by the foregoing general formulas (7), (8) and (9) as a second component; and at least one compound selected from the group consisting of those represented by the foregoing general formulas (10), (11) and (12) as a third component.

According to an eighth aspect of the present invention, there is provided a liquid crystal composition which comprises at least one liquid crystal compound defined above as a first component; at least one compound selected from the group consisting of those represented by the foregoing general formulas (5) and (6) as a second component; and at least one compound selected from the group consisting of those represented by the foregoing general formulas (7), (8) and (9) as a third component.

According to a ninth aspect of the present invention, there is provided a liquid crystal composition which comprises at least one liquid crystal compound defined above as a first component; at least one compound selected from the group consisting of those represented by the foregoing general formulas (2), (3) and (4) as a second component; at least one compound selected from the group consisting of those represented by the foregoing general formulas (5) and (6) as a third component; and at least one compound selected from the group consisting of those represented by the foregoing general formulas (7), (8) and (9) as a fourth component.

According to a tenth aspect of the present invention, there is provided a liquid crystal composition which comprises at least one optically active compound in addition to one of the liquid crystal compositions defined above.

According to an eleventh aspect of the present invention, there is provided a liquid crystal display device constituted by the use of one of the liquid crystal compositions defined above.

The sixth and seventh aspects of the present invention relate to N-type (having negative Δε) liquid crystal compositions. The N-type liquid crystal composition can be driven according to a variety of display modes such as vertically aligned mode as disclosed in J.P. KOKAI No. Hei 2-176625 and IPS mode.

Moreover, the present inventions according to the third, fourth, fifth, eighth and ninth aspects of the present invention relate to P-type (having positive Δε) liquid crystal compositions, but an N-type compound may also be used as a component for such P-type liquid crystal compositions. This permits not only arbitrary establishment of the dielectric anisotropy value of the liquid crystal composition depending on any particular use, but also the control of other characteristic properties of the composition such as the optical anisotropy value and the elastic constant.

Preferred embodiments of the compounds of the present invention, which are represented by Formula (1) will now be detailed below.

The substituents R¹ and Y¹ of the compound of Formula (1) each independently represents an alkyl group having 1 to 20 carbon atoms, a hydrogen or halogen atom, a cyano or isocyano group, or a cyanate or isothiocyanate residue, provided that at least one of the methylene groups present in the alkyl group, which are not adjacent to each other, may be substituted with an oxygen, sulfur or nitrogen atom, a group -C ≡C-, a silylene group in which at least one hydrogen atom may be substituted with an alkyl group having 1 to 10 carbon atoms, or a vinylene group, that at least one of the hydrogen atoms present in the alkyl group may be substituted with a fluorine or chlorine atom or that these alkyl groups may be optically active ones.

Specific examples of the alkyl group represented by R¹ and Y¹ in Formula (1) include saturated alkyl groups simply consisting of carbon and hydrogen atoms, haloalkyl groups, alkoxy or haloalkoxy groups, alkenyl or haloalkenyl groups, alkenyloxy or haloalkenyloxy groups, alkynyl or haloalkynyl groups, alkynyloxy or haloalkynyloxy groups, alkoxyalkyl or haloalkoxyalkyl groups, alkoxyalkoxy or haloalkoxyalkoxy groups, alkanoyl or haloalkanoyl groups, alkanoyloxy or haloalkanoyloxy groups, and alkoxycarbonyl or haloalkoxycarbonyl groups.

Specific examples of R¹ and Y¹ are groups or atoms described below:
-CH₃, -C₂H₅, -C₃H₇, -C₄H₉, -C₅H₁₁, -C₆H₁₃, -C₇H₁₅, -C₈H₁₇, -C₉H₁₉, -C₁₀H₂₁, -CFH₂, -CF₂H, -CF₃, -CH₂CH₂F, -CH₂CHF₂, -CH₂CF₃, -CF₂CH₃, -CF₂CF₂H, -CHFCF₃, -CF₂CF₃, -(CH₂)₂CH₂F, -(CH₂)₂CHF₂, -(CH₂)₂CF₃, -CH₂CF₂CH₃, -CH₂CF₂CF₃, -CF₂C₂H₅, -C₃F₇, -CF₂CFHCF₃, -(CH₂)₃CH₂F, -(CH₂)₄CH₂F, -(CH₂)₅CH₂F, -CF₂C₃H₇, -CH₂CF₂C₂H₅, -(CH₂)₂CF₂CH₃, -CF₂C₄H₉, -CH₂CF₂CH₃, -(CH₂)₂CF₂C₂H₅, -(CH₂)₃CF₂CH₃, -OCH₃, -OC₂H₅, -OC₃H₇, -OC₄H₉, -OC₅H₁₁, -OC₆H₁₃, -OC₇H₁₅, -OC₈H₁₇, -OC₉H₁₉, -OC₁₀H₂₁, -OCFH₂, -OCF₂H, -OCF₃, -OCH₂CH₂F, -OCH₂CHF₂, -OCH₂CF₃, -OCF₂CH₃, -OCF₂CF₂H, -OCHFCF₃, -OCF₂CF₃, -O(CH₂)₂CH₂F, -O(CH₂)₂CHF₂, -O(CH₂)₂CF₃, -OCH₂CF₂CH₃, -OCH₂CF₂CF₃, -OCF₂C₂H₅, -OC₃F₇, -OCF₂CFHCF₃, -O(CH₂)₃CH₂F, -O(CH₂)₄CH₂F, -O(CH₂)₅CH₂F, -OCF₂C₃H₇, -OCH₂CF₂C₂H₅, -O(CH₂)₂CF₂CH₃, -OCF₂C₄H₉, -OCH₂CF₂CH₃, -O(CH₂)₂CF₂C₂H₅, -O(CH₂)₃CF₂CH₃, -CH=CH₂, -CH=CHCH₃, -CH=CHC₂H₅, -CH=CHC₃H₇, -CH=CHC₄H₉, -CH₂CH=CHCH₃, -CH₂CH=CHC₂H₅, -CH₂CH=CHC₃H₇, -CH₂CH=CHC₄H₉, -(CH₂)₂CH=CHCH₃, -(CH₂)₂CH=CHC₂H₅, -(CH₂)₂CH=CHC₃H₇, -(CH₂)₂CH=CHC₄H₉, -CH₂CH=CH₂, -(CH₂)₂CH=CH₂, -(CH₂)₃CH=CH₂, -(CH₂)₄CH=CH₂, -(CH₂)₅CH=CH₂, -CH=CHCH=CH₂, -CH=CHCH₂CH=CH₂, -CH=CH(CH₂)₂CH=CH₂, -CH=CH(CH₂)₃CH=CH₂, -CH₂CH=CH(CH₂)₂CH=CH₂, -(CH₂)₂CH=CH(CH₂)₂CH=CH₂, -CF=CF₂, -CH=CF₂, -CH=CHF, -CH₂CF=CF₂, -CH₂CH=CHF, -(CH₂)₂CH=CF₂, -(CH ₂)₂CH=CHF, -(CH₂)₃CH=CF₂, -(CH₂)₃CH=CHF, -(CH₂)₄CH=CF₂, -(CH₂)₄CH=CHF, -(CH₂)₅CH=CF₂, -(CH₂)₅CH=CHF, -CH=CHCH₂CH₂F, -CH=CHCH₂CH₂Cl, -CH=CHCH₂CHF₂, -CH=CHCH₂CF₃, -CH=CH(CH₂)₂CH₂F, -CH=CH(CH₂)₂CHF₂, -CH=CH(CH₂)₂CF₃, -CH=CH(CH₂)₃CH₂F, -CH=CH(CH₂)₃CHF₂, -CH=CH(CH₂)₃CF₃, -CH=CH(CH₂)₄CH₂F, -CH=CH(CH₂)₄CHF₂, -CH=CH(CH₂)₄CF₃, -CH₂CH=CH(CH₂)₂CH₂F, -CH₂CH=CHCH₂CH₂F, -(CH₂)₂CH=CH(CH₂)₂CH₂F, -(CH₂)₂CH=CHCH₂CH₂F, -CH=CHCH₂CH=CF₂, -CH=CH(CH₂)₂CH=CF₂, -CH=CH(CH₂)₂CF=CF₂, -CH=CH(CH₂)₃CHFCH₃, -OCH₂CH=CHCH₃, -OCH₂CH=CHC₂H₅, -OCH₂CH=CHC₃H₇, -OCH₂CH=CHC₄H₉, -O(CH₂)₂CH=CHCH₃, -O(CH₂)₂CH=CHC₂H₅, -O(CH₂)₂CH=CHC₃H₇, -O(CH₂)₂CH=CHC₄H₉, -OCH₂CH=CH₂, -O(CH₂)₂CH=CH₂, -O(CH₂)₃CH=CH₂, -O(CH₂)₄CH=CH₂, -O(CH₂)₅CH=CH₂, -OCH₂CH=CH(CH₂)₂CH=CH₂, -O(CH₂)₂CH=CH(CH₂)₂CH=CH₂, -OCH₂CF=CF₂, -OCH₂CH=CF₂, -OCH₂CF=CHF, -O(CH₂)₂CH=CF₂, -O(CH₂)₂CH=CHF, -O(CH₂)₃CH=CF₂, -O(CH₂)₃CH=CHF, -O(CH₂)₄CH=CF₂, -O(CH₂)₄CH=CHF, -O(CH₂)₅CH=CF₂, -O(CH₂)₅CH=CHF, -OCH₂CH=CHCH₂CH₂F, -O(CH₂)₂CH=CH(CH₂)₂CH₂F, -O(CH₂)₂CH=CHCH₂CH₂F, -C ≡CH, -C≡CCH₃, -C≡ CC₂H₅, -C ≡CC₃H₇, -C ≡CC₄H₉, -CH₂C≡CH, -CH₂C ≡CCH₃, -CH₂C ≡CC₂H₅, -CH₂C≡CC₃H₇, -CH₂C≡CC₄H₉, -(CH₂)₂C ≡CH, - (CH₂)₂C ≡CCH₃, -(CH₂)₂C -CC₂H₅, -(CH₂)₂C ≡CC₃H₇, -(CH₂)₂C ≡CC₄H₉, -(CH₂)₃C ≡CH, -(CH₂)₃C≡ CCH₃, -(CH₂)₃C=CC₂H₅, -(CH₂)₃C ≡CC₃H₇, -(CH₂)₃C≡CC₄H₉, -C ≡CCF₃, -C≡CC₂F₅, -C ≡CC₃F₇, -CH₂C≡CCF₃, -(CH₂)₂C≡CCF₃, -OCH₂C≡CH, -OCH₂C ≡CCH₃, -OCH₂C≡CC₂H₅, -OCH₂C≡CC₃H₇, -OCH₂C ≡CC₄H₉, -O(CH₂)₂C≡CH, -O(CH₂)₂C ≡CCH₃, -O(CH₂)₂C ≡CC₂H₅, -O(CH₂)₂C≡CC₃H₇, -O(CH₂)₂C≡ CC₄H₉, -O(CH₂)₃C-CH, -O(CH₂)₃C ≡CCH₃, -O(CH₂)₃C ≡CC₂H₅, -O(CH₂)₃C≡ CC₃H₇, -O(CH₂)₃C=CC₄H₉, -OCH₂C ≡CCF₃, -O(CH₂)₂C ≡CCF₃, -CH₂OCH₃, -CH₂OC₂H₅, -CH₂OC₃H₇, -CH₂OC₄H₉, -CH₂OC₅H₁₁, -(CH₂)₂OCH₃, -(CH₂)₂OC₂H₅, -(CH₂)₂OC₃H₇, -(CH₂)₂OC₄H₉, -(CH₂)₂OC₅H₁₁, -(CH₂)₃OCH₃, -(CH₂)₃OC₂H₅, -(CH₂)₃OC₃H₇, -(CH₂)₃OC₄H₉, -(CH₂)₃OC₅H₁₁, -(CH₂)₄OCH₃, -CF₂OCH₃, -CF₂OC₂H₅, -CF₂OC₃H₇, -CF₂OC₄H₉, -CF₂OC₅H₁₁, -CH₂OCF₃, -CH₂OC₂F₅, -OCH₂OCH₃, -OCH₂OC₂H₅, -OCH₂OC₃H₇, -OCH₂OC₄H₉, -OCH₂OC₅H₁₁, -O(CH₂)₂OCH₃, -O(CH₂)₂OC₂H₅, -O(CH₂)₂OC₃H₇, -O(CH₂)₂OC₄H₉, -O(CH₂)₂OC₅H₁₁, -O(CH₂)₃OCH₃, -O(CH₂)₃OC₂H₅, -O(CH₂)₃OC₃H₇, -O(CH₂)₃OC₄H₉, -O(CH₂)₃OC₅H₁₁, -O(CH₂)₄OCH₃, -OCH₂OCF₃, -OCH₂OC₂F₅, -COCF₃, -COCHF₂, -COC₂F₅, -COC₃F₇, -COCH₂CF₃, -OCOCH₃, -OCOC₂H₅, -OCOC₃H₇, -OCOC₅H₁₁, -OCOC₇H₁₅, -OCOCF₃, -OCOCHF₂, -OCOC₂F₅, -OCOC₃F₇, -OCOCH₂CF₃, -COOCH₃, -COOC₂H₅, -COOC₃H₇, COOC₄H₉, -COOC₅H₁₁, -COOCH(CH₃)C₆H₁₃, -COOCH₂F, -COOCH₂CF₃, -COO(CH₂)₂CF₃, -COO(CH₂)₃CF₃, -COO(CH₂)₄CH₂F, hydrogen or halogen atoms, cyano or isocyano groups, and cyanate or isothiocyanate residues.

If paying attention, in particular, to the physicochemical stability of the compound of Formula (1), R¹ and Y¹ are preferably saturated alkyl groups simply consisting of carbon and hydrogen atoms, haloalkyl groups, alkoxy or haloalkoxy groups, alkoxyalkyl or haloalkoxyalkyl groups, alkoxyalkoxy or haloalkoxyalkoxy groups, alkanoyl or haloalkanoyl groups, alkanoyloxy or haloalkanoyloxy groups, alkoxycarbonyl or haloalkoxycarbonyl groups, hydrogen or halogen atoms, cyano or isocyano groups, and cyanate residues.

If it is desired to further increase the absolute value of the negative dielectric anisotropy of the compound of Formula (1), R¹ and Y¹ are preferably substituents having a low polarity such as saturated alkyl groups simply consisting of carbon and hydrogen atoms, alkoxy groups, alkenyl groups, alkenyloxy groups, alkynyl groups, alkynyloxy groups, alkoxyalkyl groups, alkoxyalkoxy groups, alkanoyl groups, alkanoyloxy groups, alkoxycarbonyl groups, and hydrogen atom.

The substituents X¹, X², X³ and X⁴ present on the compound of Formula (1) are as follows: a single bond, -(CH₂)₂-, -CH=CH-, -C≡C-, -COO-, -OCO-, -CH₂O-, -OCH₂-, -(CH₂)₄-, -(CH₂)₃O-, -O(CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, -CF₂CH₂-, -(CF₂)₂-, -(CF₂)₄-, -(CH₂)₂COO-, -OCO(CH₂)₂-, -CH=CHCOO-, -OCOCH=CH-, -CH=CHC ≡C-, and -C≡CCH=CH-.

If paying attention, in particular, to the physicochemical stability of the compound of Formula (1), the preferred substituents X¹, X², X³ and X⁴ are as follows:

Single bond, -(CH₂)₂-, -COO-, -OCO-, -CH₂O-, -OCH₂-, -(CH₂)₄-, -(CH₂)₃O-, -O(CH₂)₃-, -CF₂O-, -OCF₂-, -CH₂CF₂-, -CF₂CH₂-, -(CF₂)₂-, -(CF₂)₄-, -(CH₂)₂COO-, and -OCO(CH₂)₂-.

At least one of the rings A¹, A², A³, A⁴ and A⁵ of the compound represented by Formula (1) is a 2,3-Difluorocyclohexa-1, 3-diene-1,4-diyl group, but examples of other preferred rings may include bicyclo[1.1.1]pentane-1,3-diyl, trans-cyclohexane-1,4-diyl, cyclohexa-1-ene-1,4-diyl and 1,4-phenylene, provided that the carbon atoms constituting these rings may be substituted with a nitrogen, oxygen or sulfur atom and that the hydrogen atoms present on the rings may likewise be substituted with a halogen atom or a cyano group.

Specific examples thereof include 1-cyano-trans-cyclohexane-1, 4-diyl, 2,2-difluoro-trans-cyclohexane-1,4-diyl, 2-fluorocyclohexa-1-ene-1,4-diyl, 2,3-difluorobenzene-1,4-diyl and 2,2-dichlorobicyclo[1.1.1]pentane-1,3-diyl.

Examples of the rings whose hydrogen atoms present thereon may be substituted with a fluorine or chlorine atom or a cyano group are as follows:
Bicyclo[1.1.1]pentane-1,3-diyl, trans-cyclohexane-1,4-diyl, cyclohexa-1-ene-1,4-diyl, 1,4-phenylene, 1,3-dioxane-2,5-diyl, pyrimidine-2,5-diyl, pyridine-2,5-diyl, pyrazine-2,5-diyl, pyridazine-3,6-diyl, tetrahydropyrane-2,5-diyl, 1,3-dithian-2,5-diyl, 1,3-oxathian-2,5-diyl, and bicyclo[2.2.2]octane-1,4-diyl.

If paying attention, in particular, to the physicochemical stability of the compound of Formula (1), preferred rings A¹, A², A³, A⁴ and A⁵ include 2,3-difluorocyclohexa-1,3-diene-1,4-diyl, but preferred rings A¹, A², A³, A⁴ and A⁵ other than the foregoing are as follows:
1-Cyano-trans-cyclohexane-1,4-diyl, 2,2-difluoro-transcyclohexane-1,4-diyl, 2-fluorocyclohexa-1-ene-1,4-diyl, 2,3-difluorobenzene-1,4-diyl, and 2,2-dichlorobicyclo[1.1.1]pentane-1, 3-diyl.

Examples of the rings whose hydrogen atoms present thereon may be substituted with a fluorine or chlorine atom or a cyano group are as follows:
Bicyclo[1.1.1]pentane-1,3-diyl, trans-cyclohexane-1,4-diyl, cyclohexa-1-ene-1,4-diyl, 1,4-phenylene, 1,3-dioxane-2,5-diyl, pyrimidine-2,5-diyl, pyridine-2,5-diyl, pyridazine-3,6-diyl, tetrahydropyrane-2,5-diyl, 1,3-dithian-2,5-diyl, and 1,3-oxathian-2,5-diyl.

If it is desired to further increase the absolute value of the negative dielectric anisotropy of the compound of Formula (1), preferred rings A¹, A², A³, A⁴ and A⁵ include 2,3-difluorocyclohexa-1,3-diene-1,4-diyl, but preferred rings A¹, A², A³, A⁴ and A⁵ other than the foregoing are as follows:
1-Cyano-trans-cyclohexane-1,4-diyl, 2,2-difluoro-transcyclohexane-1,4-diyl, 2-fluorocyclohexa-1-ene-1,4-diyl, 2,3-difluorobenzene-1,4-diyl, and 2,2-dichlorobicyclo[1.1.1]pentane-1, 3-diyl.

Examples of the rings whose hydrogen atoms present thereon may be substituted with a fluorine or chlorine atom or a cyano group are as follows:
Trans-cyclohexane-1,4-diyl, cyclohexa-1-ene-1,4-diyl, pyridazine-3,6-diyl, and tetrahydropyrane-2,5-diyl.

The compounds according to the first aspect of the present invention and preferred embodiments thereof have negative dielectric anisotropy values whose absolute values are large. Therefore, a liquid crystal composition having a negative dielectric anisotropy value can be prepared by mainly using the compounds of the present invention. This liquid crystal composition is useful in the preparation of devices which make use of a liquid crystal composition having negative dielectric anisotropy, including, for instance, vertically aligned mode as disclosed in J.P. KOKAI No. Hei 2-176625 and IPS mode. Moreover, the dielectric anisotropy value can appropriately be established by the use of a mixture of the compound of the present invention with other liquid crystal compounds or compositions to thus expand the area of applications of these other liquid crystal compounds or compositions.

The compounds according to the first aspect of the present invention and preferred embodiments thereof have small elastic constants and the temperature-dependency of the constant is also low. Therefore, the use of the compounds of the present invention would permit the preparation of novel liquid crystal compositions for a low driving voltage.

The compounds according to the first aspect of the present invention and preferred embodiments thereof exhibit good miscibility with other liquid crystal compounds. For this reason, if the compounds of the present invention are incorporated into liquid crystal compositions, any problem such as separation of compounds rarely arises. In addition, a large amount of the compound of the present invention can be incorporated into a liquid crystal composition and accordingly, the excellent characteristics of the compound of the present invention can intensively be reflected in the resulting liquid crystal composition.

The compounds according to the first aspect of the prtesent invention and preferred embodiments thereof permit appropriate design of the structure of the compounds such that they have a desired level of the optical anisotropy value. In other words, the compound should be designed so as to have rings including a large number of sites carrying resonance structures such as aromatic rings, if it is necessary to use a compound having a particularly high optical anisotropy value. On the other hand, if it is necessary to use a compound having a low optical anisotropy value, the compound should be designed so as to have rings including a large number of sites free of any resonance structure such as trans-cyclohexane-1,4-diyl ring.

If describing this more fully, the compound of Formula (1) according to the present invention may have a desired level of the optical anisotropy value by appropriately selecting the substituents R¹, Y¹, X¹, X², X³, X⁴, A¹, A², A³, A⁴, A⁵, a¹, a², a³, a⁴ and a⁵. More specifically, the optical anisotropy value may be controlled by appropriately selecting the rings A¹, A², A³, A⁴ and A⁵. In other words, the selection of a ring or rings each including a large number of sites carrying resonance structures such as aromatic rings permits the preparation of a compound having particularly high optical anisotropy, while the selection of a ring or rings each including a large number of sites free of any resonance structure such as transcyclohexane-1,4-diyl ring permits the preparation of a compound having low optical anisotropy.

The degree of freedom in the design of a liquid crystal panel would widely be expanded by arbitrarily selecting the optical anisotropy value.

All of the compounds according to the first aspect of the present invention and preferred embodiments thereof have low viscosities and therefore, they do not undesirably increase the overall viscosity of the resulting liquid crystal composition even if a large amount of the compound is incorporated into the composition. In addition, the compound of the present invention exhibits very low temperature-dependency of the viscosity, in particular, at a low temperature. The use of the liquid crystal compound having such an excellent viscosity permits the preparation of a liquid crystal composition having high speed responsibility.

All of the compounds according to the first aspect of the present invention and preferred embodiments thereof are physicochemically stable and accordingly, the liquid crystal compositions prepared using them have high specific resistance and a high voltage-holding ratio. In other words, the compounds are highly stable against external factors such as ultraviolet rays and heat and have physicochemical stability sufficient for use as ingredients of practically used liquid crystal compositions.

The phase transition temperature of the compounds according to the first aspect of the present invention and preferred embodiments thereof can be controlled by appropriately selecting the number of rings attached to each compound. In other words, it would be sufficient to select a compound carrying not less than 4 rings when it is desired for the achievement of a particularly high clearing point, a compound having 3 rings when it is desired for the achievement of a medium clearing point, and a compound having 2 rings when it is desired for the achievement of a particularly low clearing point.

If describing this more fully, the phase transition temperature of the compounds of Formula (1) according to the present invention can be controlled by appropriately selecting the values of a¹, a², a³, a⁴ and a⁵. More specifically, it would be sufficient to select a compound carrying not less than 4 rings and a¹+a²+a³+a⁴+a⁵ ≧4 when it is desired for the achievement of a particularly high clearing point; a compound having 3 rings and a¹+a²+a³+a⁴+a⁵=3 when it is desired for the achievement of a medium clearing point; and a compound having 2 rings and a¹+a²+a³+a⁴+a⁵=2 when it is desired for the achievement of a particularly low clearing point.

As has been discussed above, the compounds of the first aspect of the present invention and the preferred embodiments thereof have various characteristic properties favorable for use as electro-optical display devices. The use of the compounds of the present invention permits the preparation of novel liquid crystal compositions having good characteristics. In addition, the present invention also permits the production of liquid crystal compounds having desired properties by appropriately designing the structure thereof depending on each particular use as well as liquid crystal composition and liquid crystal display devices using the compounds.

For instance, the compound of the present invention is characterized by having a large negative dielectric anisotropy value, but the compound can not only be used in a liquid crystal composition having a negative dielectric anisotropy value, but also can be used to control the dielectric anisotropy value and other characteristics of a composition having a positive dielectric anisotropy value by the addition of the former to the latter.

If describing this more fully, the compound of the present invention and the liquid crystal composition including the same can be used in various display modes which make use of compounds or compositions having negative dielectric anisotropy values (such as the vertically aligned mode as disclosed in J.P. KOKAI No. Hei 2-176625, IPS mode, ECB mode (for instance, HAN mode or DAP mode), DS mode, GH mode or PC mode), in particular, the vertically aligned mode as disclosed in J.P. KOKAI No. Hei 2-176625 and IPS mode. In addition to the foregoing, the compound and composition of the present invention can also be used for the improvement or control of various characteristics (for instance, dielectric anisotropy, elastic constant, optical anisotropy, viscosity, and/or physicochemical stability) of liquid crystal compositions used for various display modes (such as TN mode, STN mode, or TN-based AM mode (e.g., TFT mode or MIM mode)) which make use of compounds or compositions having positive dielectric anisotropy values.

The liquid crystal composition of the present invention will now be detailed below. The liquid crystal composition of the present invention preferably comprises 0.1 to 99.9% by weight of at least one compound as defined in the first aspect of the present invention and the preferred embodiments thereof in order to impart excellent characteristic properties to the composition.

More specifically, the liquid crystal composition of the present invention comprises a compound selected from the group consisting of those represented by Formulas (2) to (12) in an appropriate amount depending on each particular purpose, in addition to at least one of the compounds of the first aspect of the present invention and the preferred embodiments thereof as a first component.

The following are examples of preferred compounds represented by Formulas (2) to (4) which can be used in the liquid crystal composition of the present invention (in these compounds, R² and Y² are the same as those defined above):

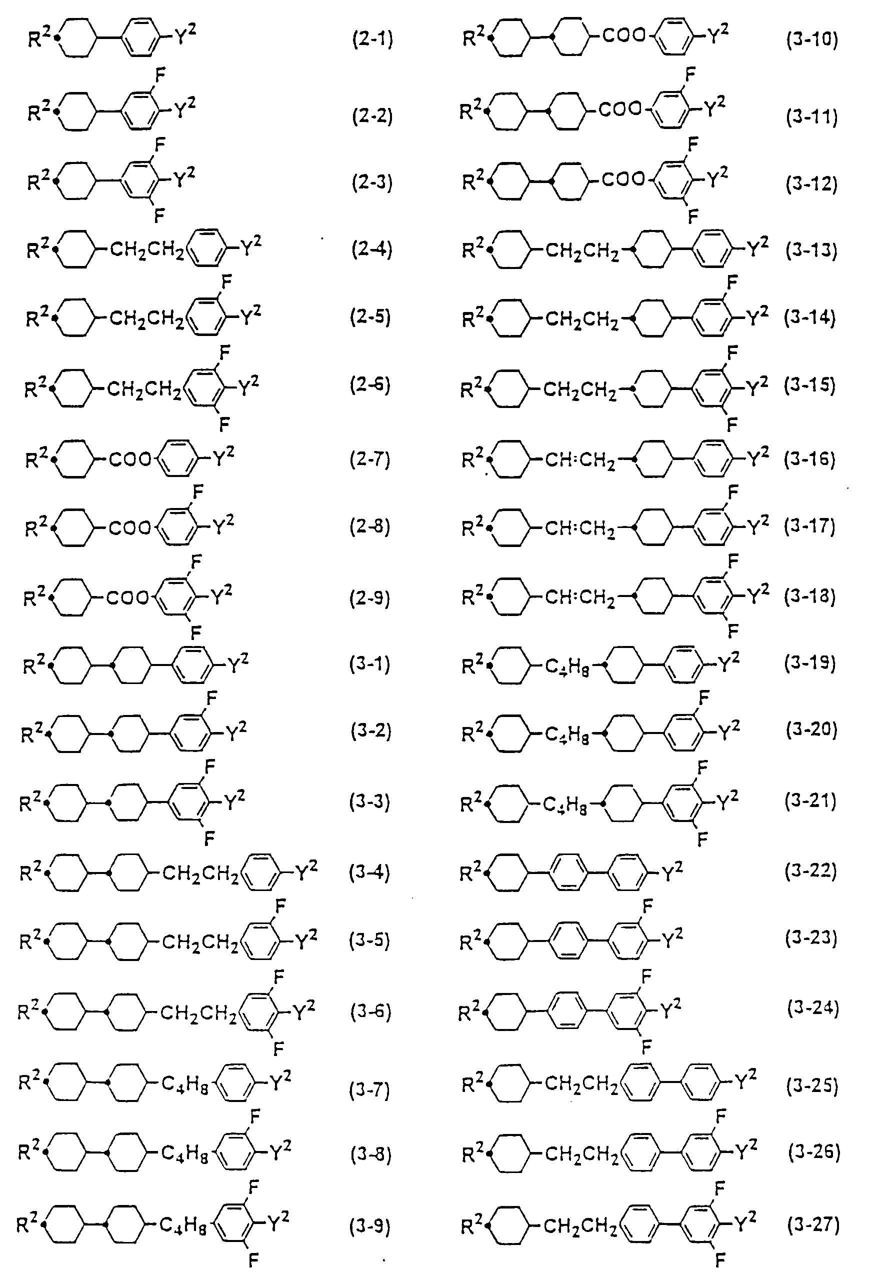

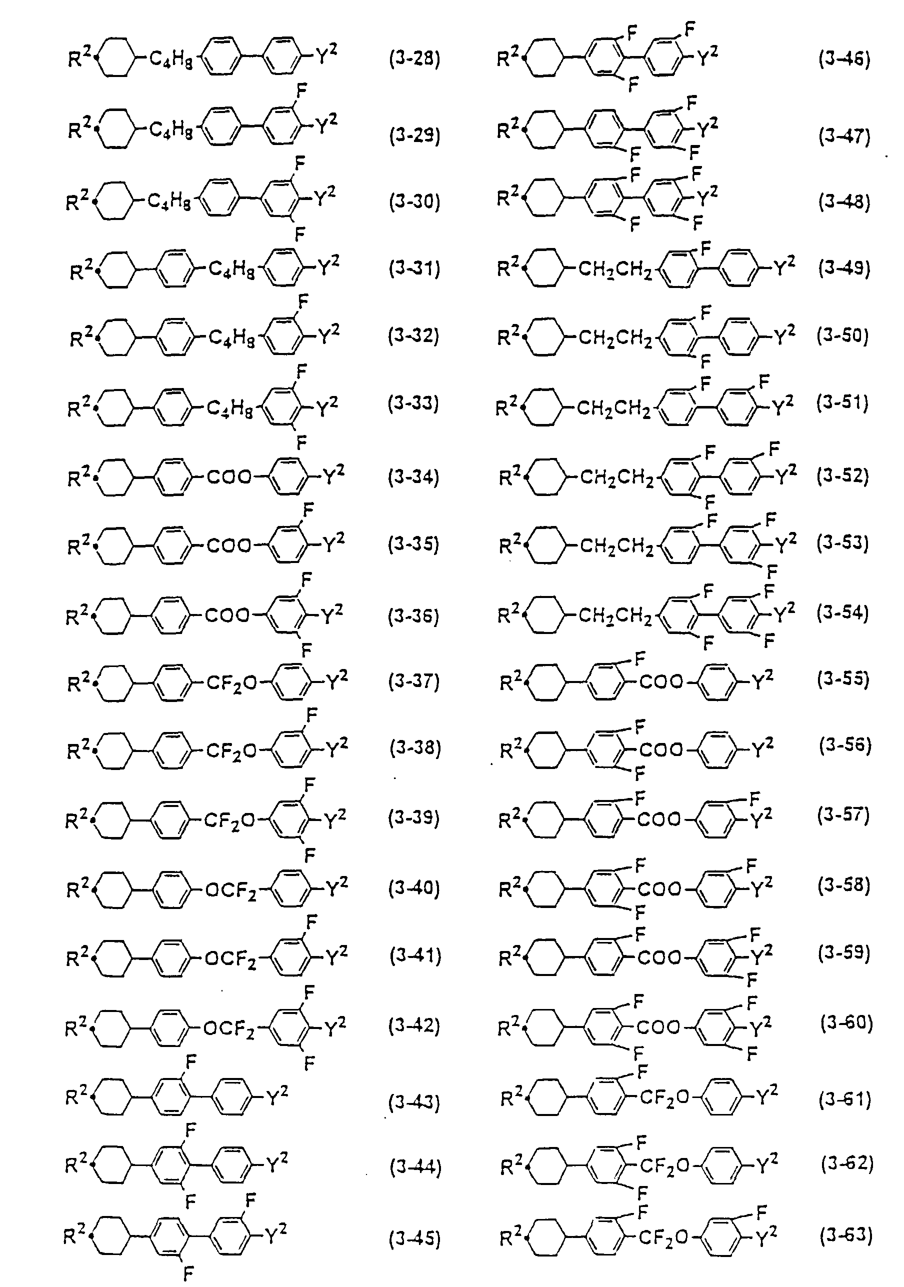

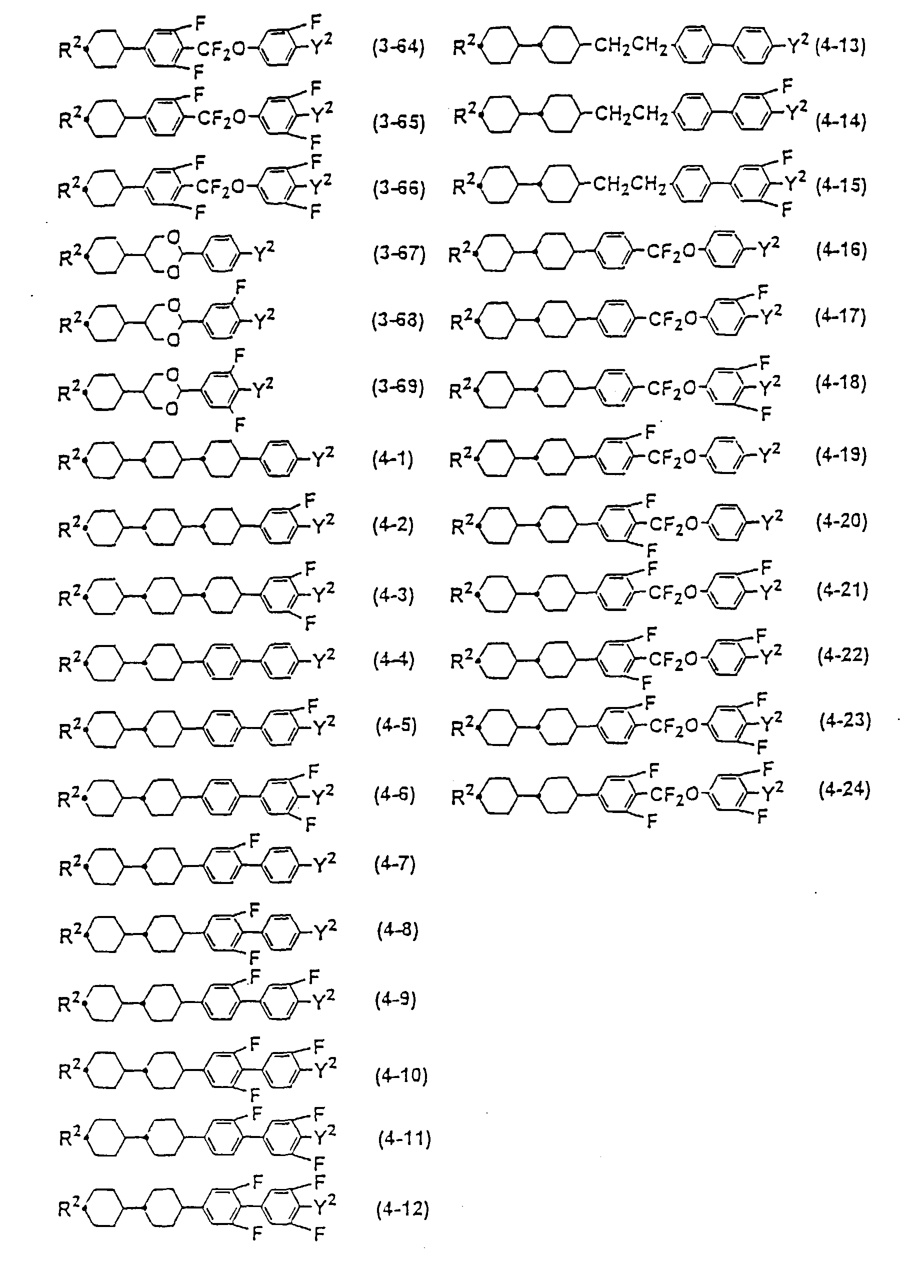

The compounds represented by Formulas (2) to (4) have positive dielectric anisotropy values, are quite excellent in thermal and chemical stability and are particularly preferably used when preparing liquid crystal compositions used in TFT's which require high reliability such as a high voltage-holding ratio or a high specific resistance.

The amount of the compounds of Formulas (2) to (4) used when preparing a liquid crystal composition used in TFT's ranges from 0.1 to 99.9% by weight, preferably 10 to 97% by weight and more preferably 40 to 95% by weight based on the total weight of the composition.

In addition, the liquid crystal composition may further comprise the compounds of Formulas (7) to (9) in order to control the viscosity of the composition.

The compounds represented by Formulas (2) to (4) can likewise be used when preparing a liquid crystal composition for STN's or TN's. In this case, they are preferably used in an amount of not more than 50% by weight.

The following are examples of preferred compounds represented by Formulas (5) to (6) which can be used in the liquid crystal composition of the present invention (in these compounds, R³, Y³ and R⁴ are the same as those defined above):

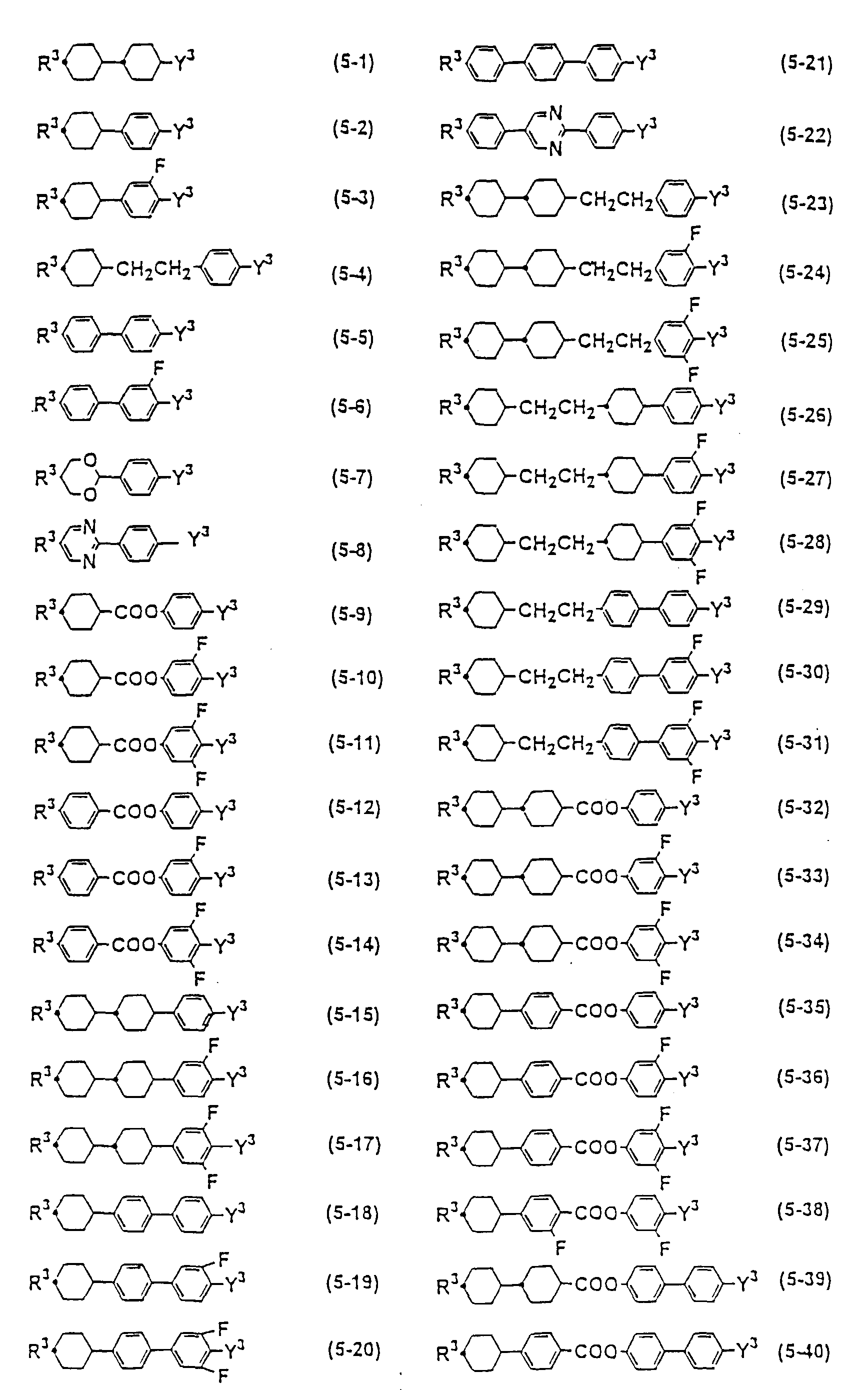

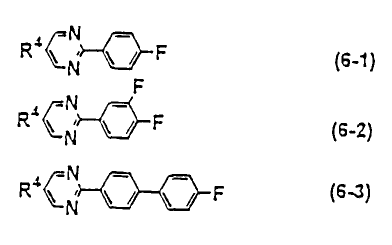

The compounds represented by Formulas (5) to (6) have large and positive dielectric anisotropy values and are used, in particular, for the purpose of reducing the threshold voltage of the resulting liquid crystal composition. Moreover, they can be used for controlling the optical anisotropy value and for expanding the nematic range, for instance, increasing the clearing point. Further they are also used for the improvement in the steepness of the V-T curve of the liquid crystal composition for STN's or TN's.

The compounds represented by Formulas (5) to (6) are preferred, in particular, when preparing the liquid crystal composition for STN's or TN's.

If the amount of the compound of Formula (5) or (6) used increases, the threshold voltage of the resulting liquid crystal composition is reduced and the viscosity thereof increases. Therefore, it is advantageous to use the compounds in a large amount since the resulting composition can be operated at a low voltage, so far as the composition satisfies the requirement for viscosity.

If preparing a liquid crystal composition for STN's or TN's, the amount of the compound of Formula (5) or (6) ranges from 0.1 to 99.9% by weight, preferably 10 to 97% by weight and more preferably 40 to 95% by weight based on the total weight of the composition.

The following are examples of preferred compounds represented by Formulas (7) to (9) which can be used in the liquid crystal composition of the present invention (in these compounds, R⁵ and R⁶ are the same as those defined above):

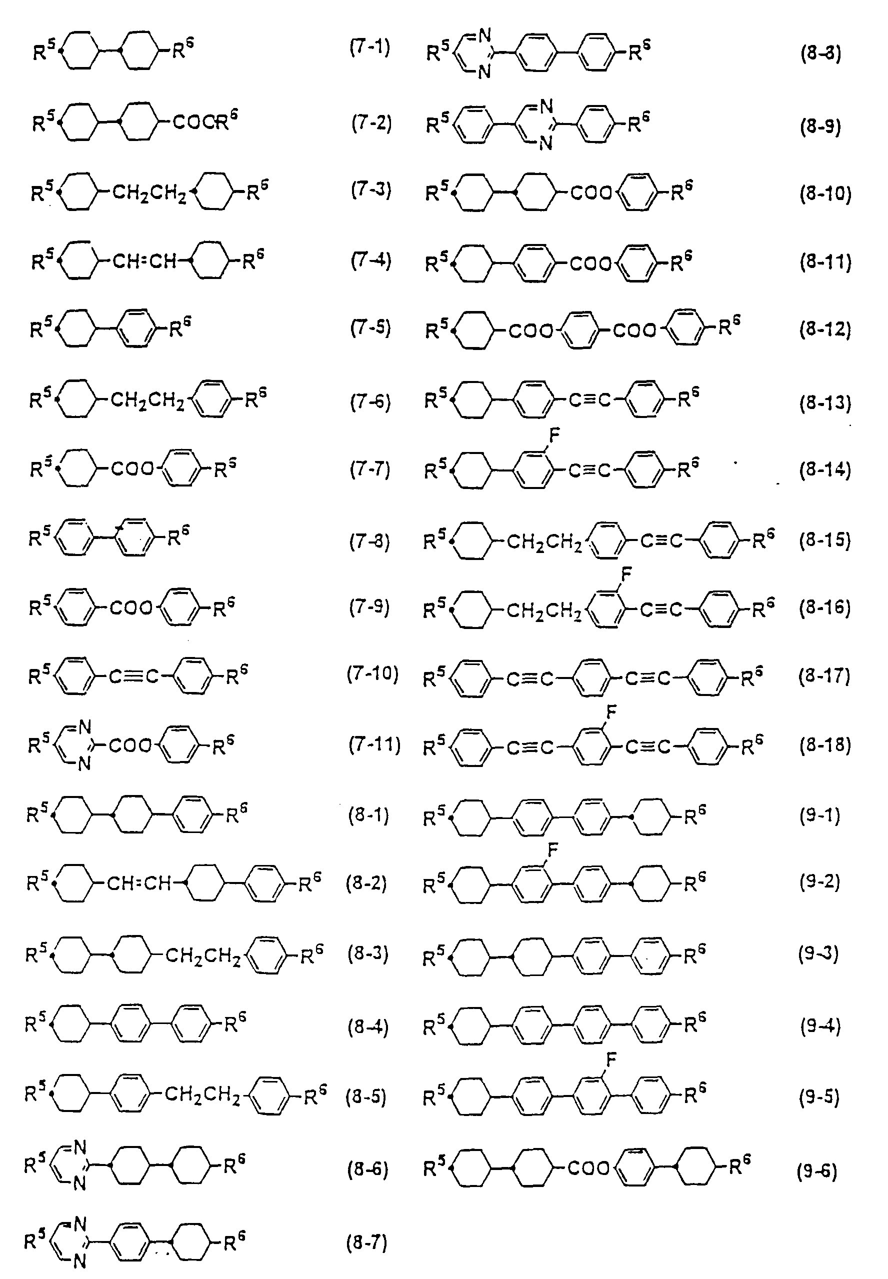

The compounds represented by Formulas (7) to (9) have dielectric anisotropy values whose absolute values are small and are almost neutral ones. The compounds of Formula (7) are mainly used for controlling the viscosity or optical anisotropy value of the resulting liquid crystal composition, while the compounds of Formula (8) and (9) are used for expanding the nematic range, for instance, increasing the clearing point or for controlling the optical anisotropy value.

If the amount of the compounds of Formulas (7) to (9) used increases, the threshold voltage of the resulting liquid crystal composition increases and the viscosity thereof decreases. Therefore, they can be used in a large amount inasmuch as the composition satisfies the requirememt for threshold voltage. When preparing a liquid crystal composition for TFT's, the amount of the compounds of Formulas (7) to (9) is preferably not more than 40% by weight and more preferably not more than 35% by weight based on the total weight of the composition, while when preparing a liquid crystal composition for STN's or TN's, the amount thereof is preferably not more than 70% by weight and more preferably not more than 60% by weight based on the total weight of the composition.

The following are examples of preferred compounds represented by Formulas (10) to (12) which can be used in the liquid crystal composition of the present invention (in these compounds, R⁷ and R⁸ are the same as those defined above):

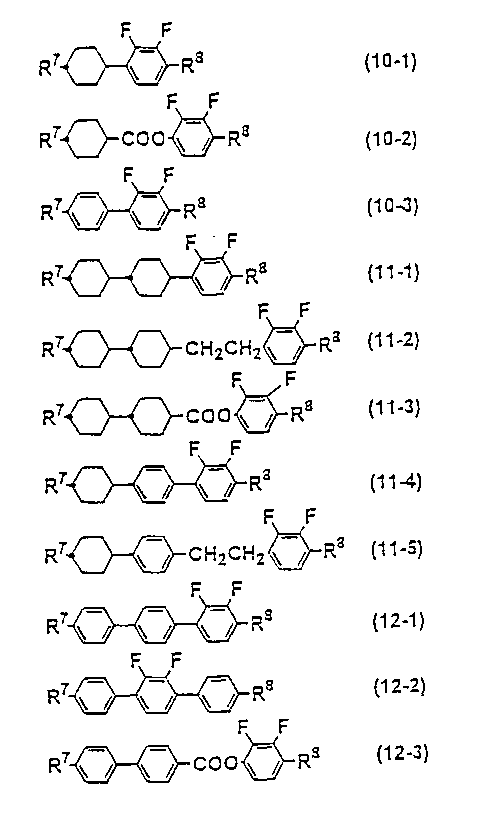

The compounds represented by Formulas (10) to (12) have negative dielectric anisotropy values like the compounds according to the first aspect of the present invention and the preferred embodiments thereof and are used as base compounds for N-type liquid crystal compositions or are used for controlling the dielectric anisotropy value of P-type liquid crystal compositions.

Moreover, the compounds of Formula (10) are bicyclic compounds and mainly used for controlling the threshold voltage, viscosities or optical anisotropy values of the resulting compositions. The compounds of Formula (11) are used for expanding the nematic range, for instanc e, increasing the clearing point or for controlling the optical anisotropy value. Further the compounds of Formula (12) are used not only for expanding the nematic range, but also for reducing the threshold voltage and for increasing the optical anisotropy value of the resulting liquid crystal composition.

The compounds of Formula (10) to (12) are mainly used in the N-type liquid crystal compositions and if the amount thereof used increases, the threshold value of the resulting liquid crystal composition is reduced, while the viscosity thereof is increased. For this reason, these compounds are desirably used in a small amount inasmuch as the liquid crystal composition satisfies the requirement for threshold voltage. However, the absolute value of the negative dielectric anisotropy thereof is not more than 5 and therefore, if the amount thereof used is less than 40% by weight, the resulting display device cannot sometimes be operated at a low voltage. When preparing a liquid crystal composition for N-type TFT's, the amount of the compounds of Formula (10) to (12) is preferably not less than 40% by weight and more preferably 50 to 95% by weight based on the total weight of the composition. Moreover, the compounds of Formula (10) to (12) are often incorporated into P-type compositions for controlling the elastic constant and the voltage-transmittance curve (V-T curve) of the resulting liquid crystal composition. In this case, the amount of the compounds of Formula (10) to (12) is preferably not more than 30% by weight based on the total weight of the composition.

In addition, the liquid crystal composition of the present invention may in general comprise an optically active compound in order to induce the formation of a helical structure of the liquid crystal composition to thus achieve a desired twist angle and prevent the occurrence of any reverse twist, except for some particular cases such as liquid crystal compositions for OCB (Optically Compensated Birefringence) mode.

Any known optically active compounds used for such purposes may be used in the present invention, but preferred are optically active compounds listed below:

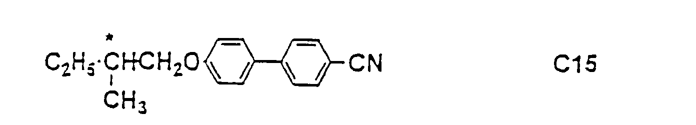

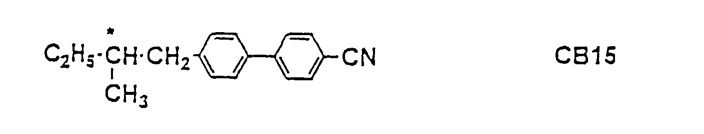

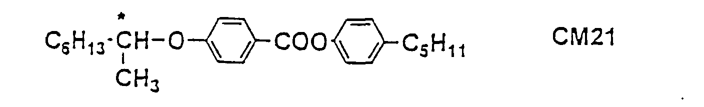

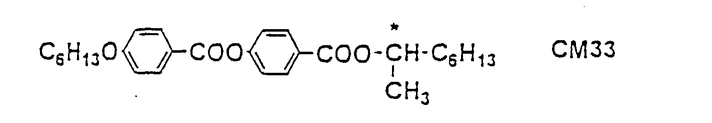

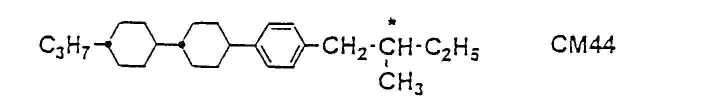

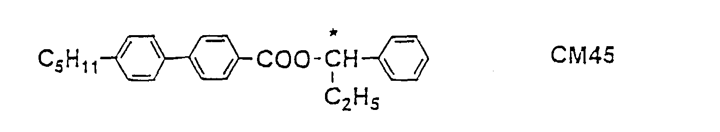

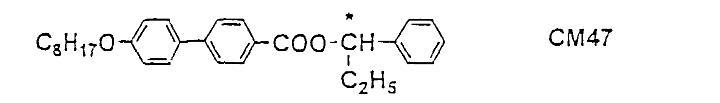

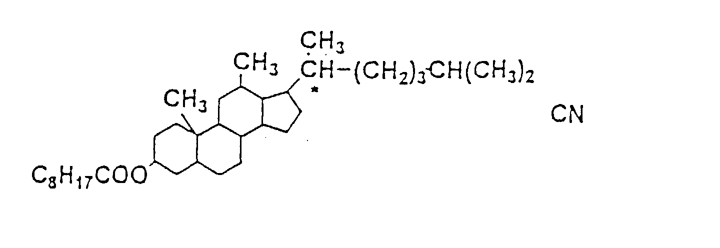

These optically active compounds are usually incorporated into the liquid crystal composition of the present invention to thus control the pitch of twist. The pitch of twist is preferably adjusted to the range of from 40 to 200 µm in case of the liquid crystal compositions for TF T's and TN's. Moreover, it is adjusted to the range of from 6 to 20 µm in case of the liquid crystal compositions for STN's and 1.5 to 4 µm for bistable TN's. Moreover, at least two optically active compounds may be added to the composition to control the temperature-dependency of the pitch.

The liquid crystal composition of the present invention may be used as the composition for GH's by addition of a dichroic dye such as merocyanine, styryl, azo, azomethine, azoxy, quinophthalone, anthraquinone, or tetrazine type ones. Alternatively, the composition of the present invention can likewise be used as a liquid crystal composition for polymer-dispersion type liquid crystal display devices (PDLCD) including NCAP's which are produced by encapsulation of a nematic liquid crystal and polymer network liquid crystal display devices (PNLCD) produced by forming a three-dimensional network polymer in the liquid crystal. Moreover, the liquid crystal composition of the present invention may be used in DS's.

The composition of the present invention can be prepared by the commonly used methods. In general, it is prepared by a method wherein various ingredients are dissolved together at a high temperature.

The compounds according to the first aspect of the present invention and the preferred embodiments thereof can easily be prepared by any known organic chemical synthesis technique. An example of the production methods will be given in the following reaction scheme 1:

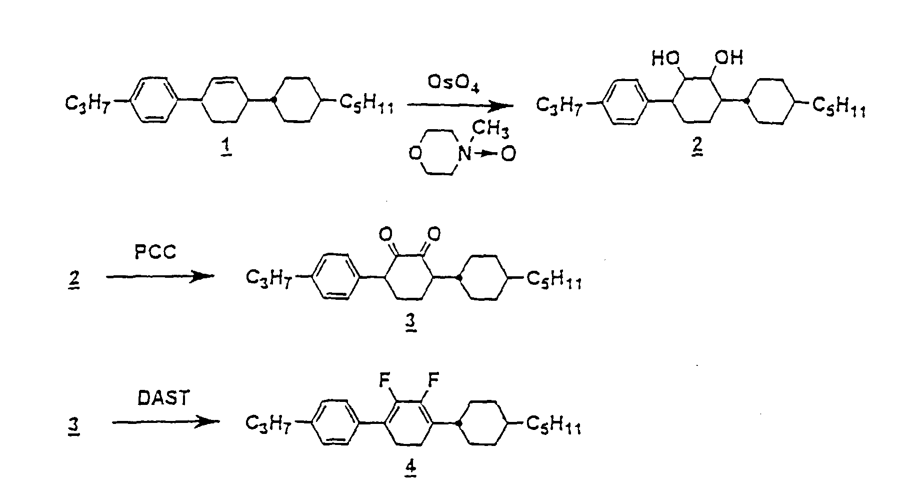

The foregoing route of synthesis will be detailed below.

The compound represented by Formula 1 is oxidized with osmium tetraoxide to thus convert it into the compound of Formula 2.

The compound represented by Formula 2 is oxidized with PCC (pyridinium chlorochromate) into the compound of Formula 3.

The compound of Formula 3 is fluorinated with DAST (Et₂NSF₃) to give the intended compound of Formula 4.

The method of preparing the compound of the present invention and the use thereof will be described below in detail with reference to the following working Examples, but the present invention is not restricted to these specific Examples at all. In the description of the following Examples, the symbol N represents the normal concentration (gram eq./l); M represents the molar concentration (mole/l); Δε(dielectric anisotropy value) of a liquid crystalline compound represents the value obtained by extrapolating the values (as measured at 25 °C) observed for the compositions comprising 85 wt% of the following mother liquid crystal A and 15 wt% of each corresponding compound; and Δn (optical anisotropy value) represents the measured value (as determined at 25 °C) for the composition comprising 85 wt% of the following mother liquid crystal B and 15 wt% of each corresponding compound.

### Composition of Mother Liquid Crystal A

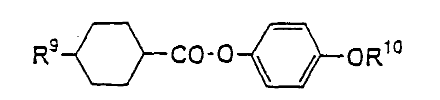

Five kinds of ester compounds represented by the foregoing general formula and whose both terminal alkyl groups (R⁹, R¹⁰) were different from each other were mixed in a ratio specified below to give the desired mother liquid crystal A.

| | | |
|---|---|---|
| R⁹ = C₃H₇, | R¹⁰ = C₄H₉ | 27.6 wt% |
| R⁹ = C₄H₉, | R¹⁰ = C₂H₅ | 20.7 wt% |
| R⁹ = C₅H₁₁, | R¹⁰ = CH₃ | 20.7 wt% |
| R⁹ = C₃H₇, | R¹⁰ = C₂H₅ | 17.2 wt% |
| R⁹ = C₅H₁₁, | R¹⁰ = C₂H₅ | 13.8 wt% |
| | | 100.0 |
| Δε = -1.5 | | |

### Composition of Mother Liquid Crystal B

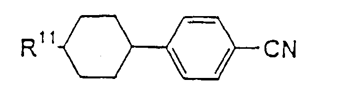

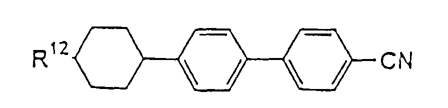

Four kinds of ester compounds represented by the foregoing general formulas and whose both terminal alkyl groups (R¹¹, R¹²) were different from each other were mixed in a ratio specified below to give the desired mother liquid crystal B.

| | |
|---|---|
| R¹¹ = C₃H₇ | 24.0 wt% |
| R¹¹ = C₅H₁₁ | 36.0 wt% |
| R¹¹ = C₇H₁₅ | 25.0 wt% |
| R¹² = C₅H₁₁ | 15.0 wt% |
| | 100.0 |
| Δn = 0.137 | |

### Example 1: Synthesis of 1-(Trans-4-n-Pentylcyclohexyl)-2,3-Difluoro-4-(4-n-Propylphenyl)Cyclohexa-1,3-Diene (4)

### First Step: Synthesis of 3-(Trans-4-n-Pentylcyclohexyl)-6-(4-n-Propylphenyl)Cyclohexane-1,2-Diol (2)

To a mixture of 3-(trans-4-n-pentylcyclohexyl)-6-(4-n-propylphenyl)cyclohexene (1) (352.6g, 1.00 mole), N-methyl morpholine-N-oxide (234.3g, 2.00 mole), acetonitrile (800 ml) and water (400 ml), there was added osmium tetraoxide (12.71g, 0.05 mole), followed by reacting them at room temperature for 20 hours. After completion of the reactio n, a saturated aqueous solution of sodium sulfite (1000 ml) was added to the reaction mixture, followed by reaction for additional one hour and extraction of the reaction mixture with ethyl acetate (3000 ml). The resulting organic phase was successively washed with a 1N dilute hydrochloric acid solution (1000 ml) and water (1000 ml) and then dried over anhydrous magnesium sulfate. The solvent was distilled off under reduced pressure, the resulting residue was purified by silica gel column chromatography (eluent: 10:1 methylene chloride/ethanol) to give the title compound, 3-(trans-4-n-pentylcyclohexyl)-6-(4-n-propylphenyl)cyclohexane-1,2-dio l (2)(246.7g, 0.638 mole; yield 64%). MS/387 (M⁺ ).

### Second Step: Synthesis of 3-(Trans-4-n-Pentylcyclohexyl)-6-(4-n-Propylphenyl)Cyclohexane-1,2-dione (3)

To a mixture of 3-(trans-4-n-pentylcyclohexyl)-6-(4-n-propylphenyl)cyclohexane-1,2-diol (2) (246.5g, 0.638 mole) prepared in the first step, silica gel (50 g) and methylene chloride (1000 ml), there was added, with ice-cooling, PCC (412.3g, 1.913 mole) over 20 minutes, followed by reacting them at room temperature for 3 hours. After completion of the reaction, the reaction solution was filtered through a Celite layer, then the resulting filtrate was washed with water (700 ml) and dried over anhydrous magnesium sulfate. The solvent was distilled off under reduced pressure, the resulting residue was purified by silica gel column chromatography (eluent: toluene) to give the title compound, 3-(trans-4-n-pentylcyclohexyl) -6-(4-n-propylphenyl) cyclohexane-1,2-dione (3) (167.3g, 0.437 mole; yield 69%). MS/383 (M ⁺ ).

### Third Step: Synthesis of 1-(Trans-4-n-Pentylcyclohexyl)-2,3-Difluoro-4-(4-n-Propylphenyl)Cyclohexa-1,3-Diene (4)

A mixture of 3-(trans-4-n-pentylcyclohexyl)-6-(4-n-propylphenyl) cyclohexane-1,2-dione (3) (167.0g, 0.436 mole) prepared in the second step and methylene chloride (600 ml) was cooled to -20°C, followed by dropwise addition of a solution of DAST (245.7g, 1.52 mole) in methylene chloride (1000 ml) at that temperature. After the dropwise addition, the mixture was reacted over two days while gradually raising the temperature up to room temperature. After completion of the reaction, a 1N sodium hydrogen carbonate aqueous solution (2000 ml) was added to the reaction solution, followed by extraction of the reaction solution with methylene chloride (2000 ml). The resulting organic phase was washed with water (2000 ml) and dried over anhydrous magnesium sulfat e. The solvent was distilled off under reduced pressure and the resulting residue was purified by silica gel column chromatography (eluent: hexane) and subsequent recrystallization (hexane) to give the title compound, 1-(trans-4-n-pentylcyclohexyl)-2, 3-difluoro-4-(4-n-propylphenyl)cyclohexa-1,3-diene (4) (30.87g, 0.0799 mole; yield 18%). MS/387 (M ⁺ );Δε=-2.0; Δn=0.139.

The following compounds can be prepared according to the method described in Example 1.

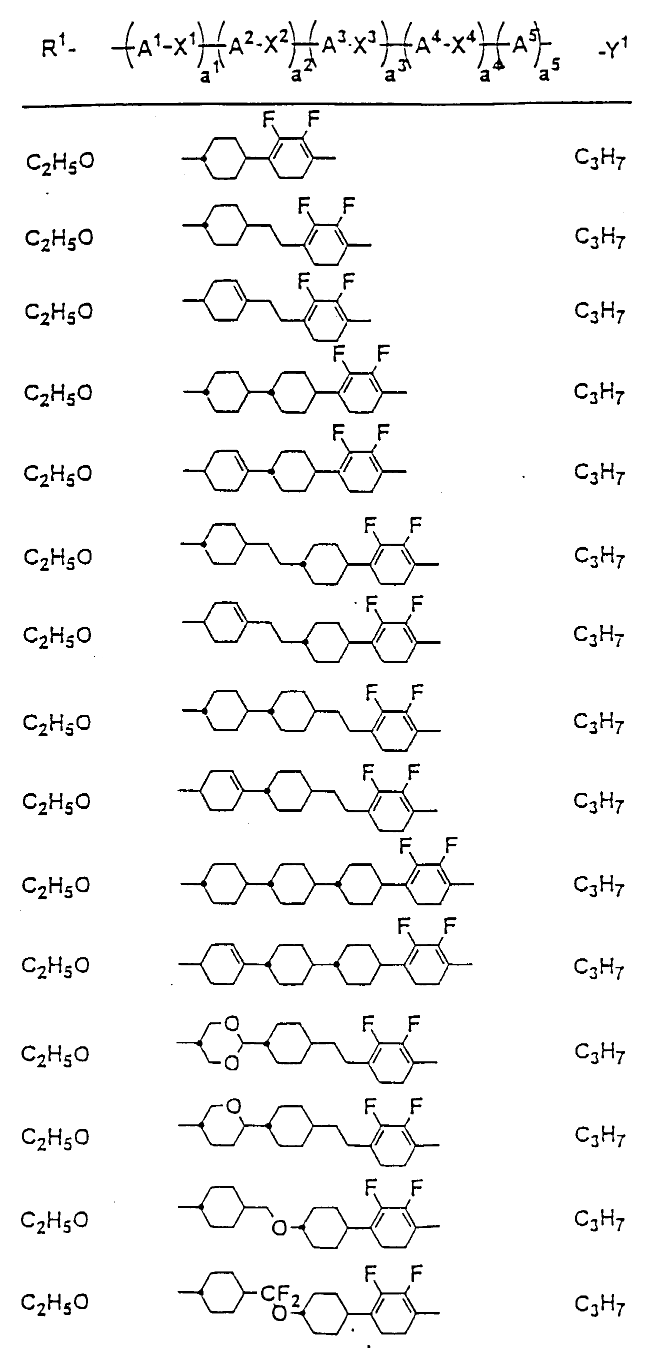

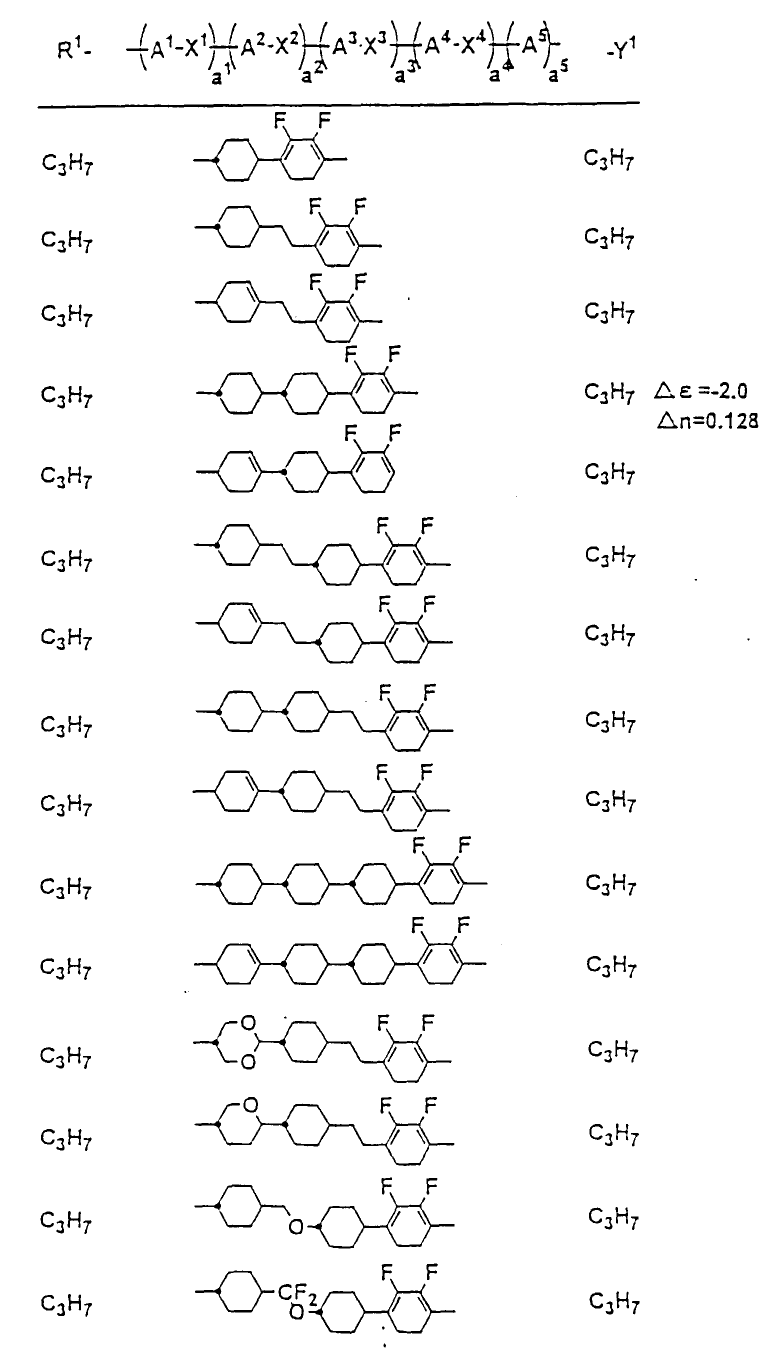

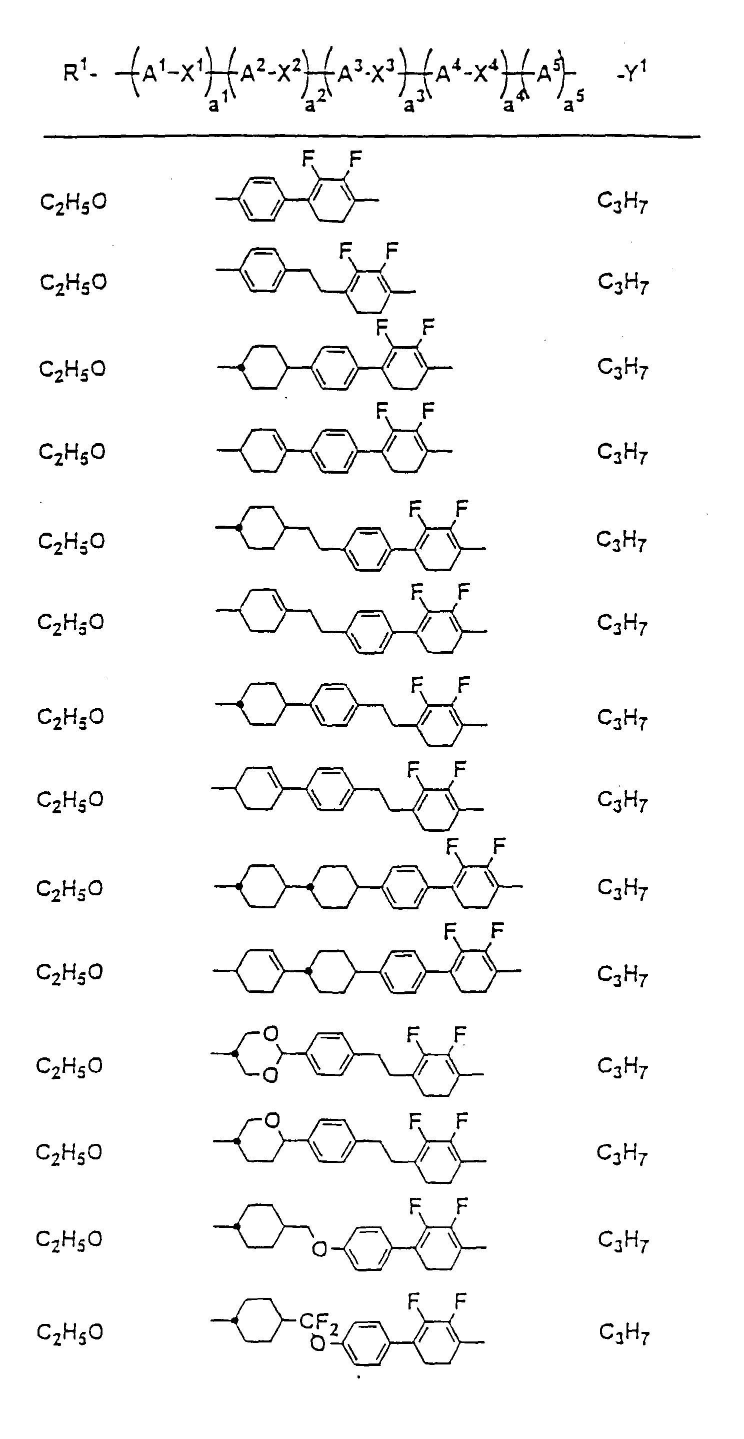

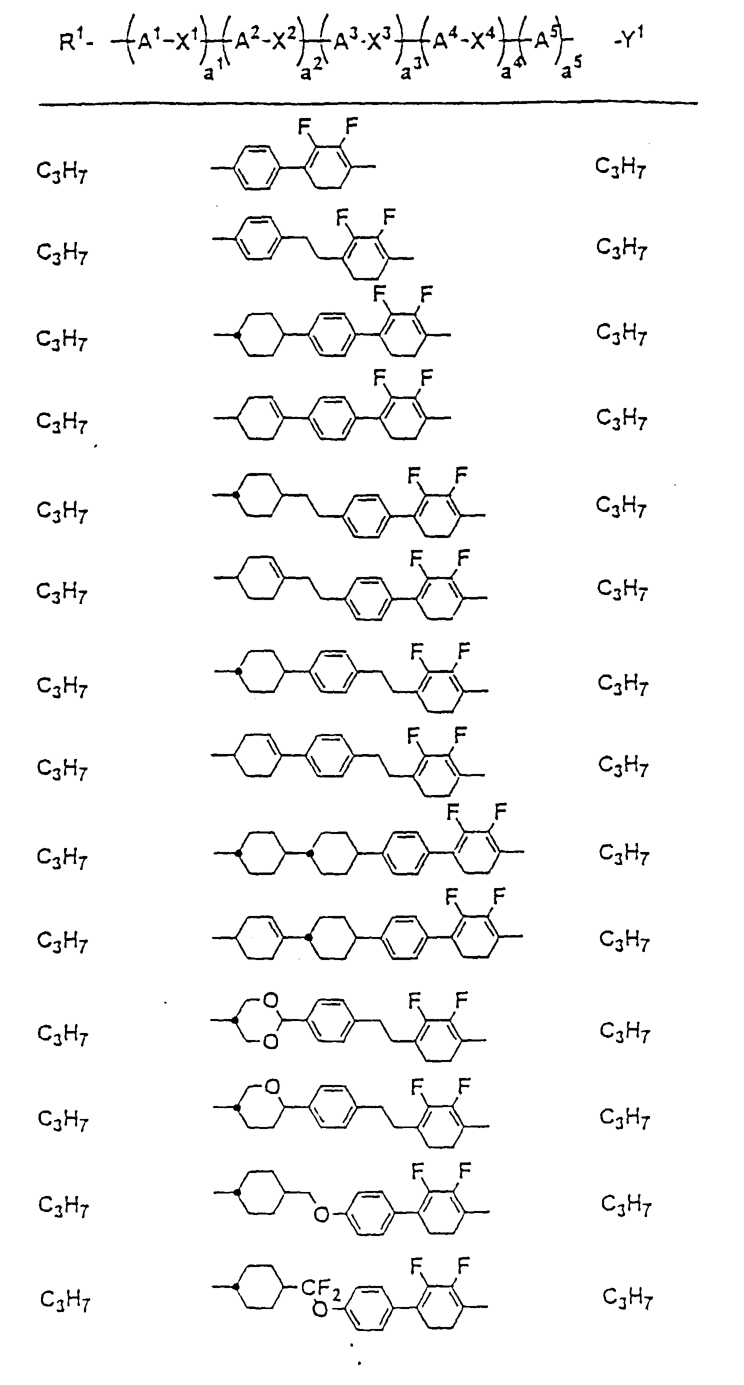

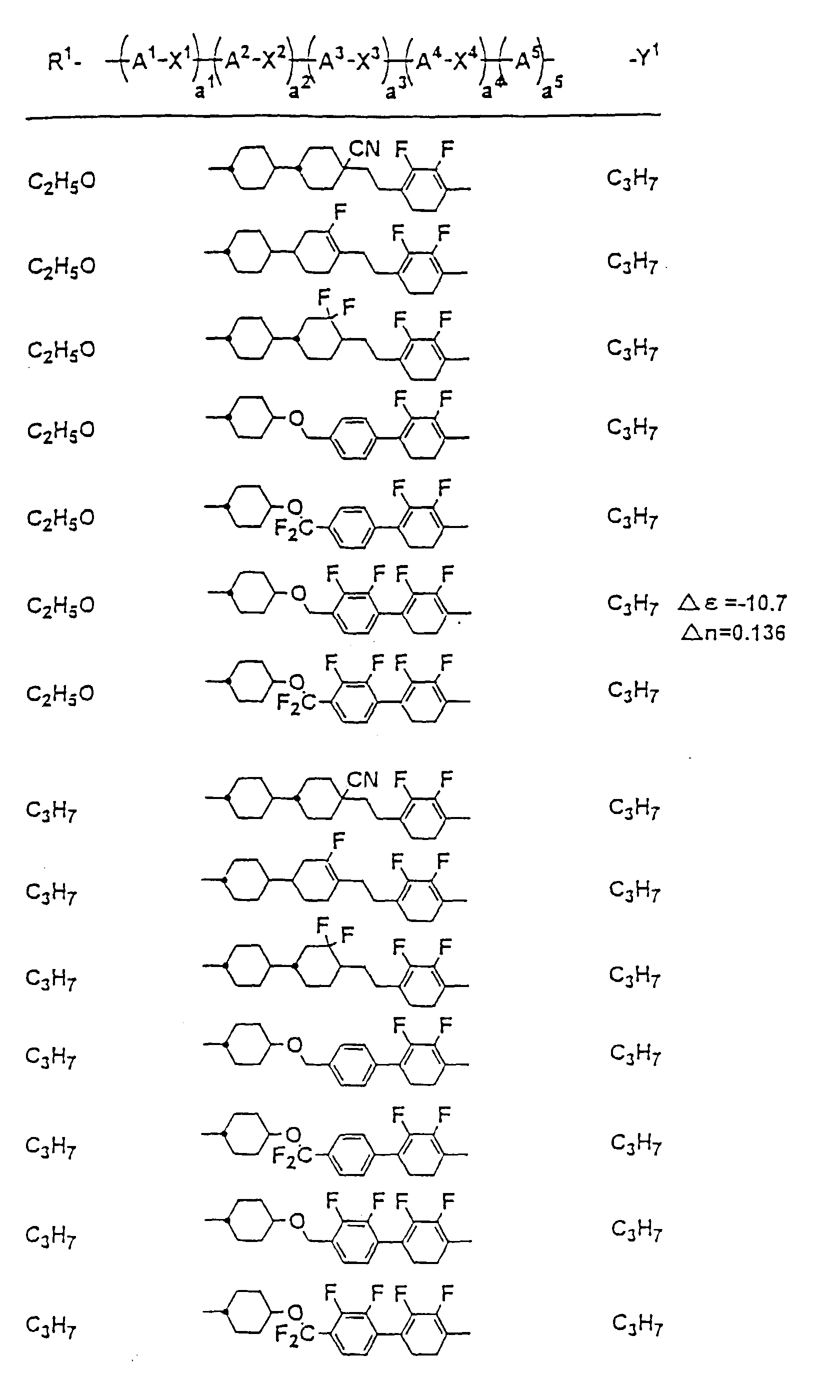

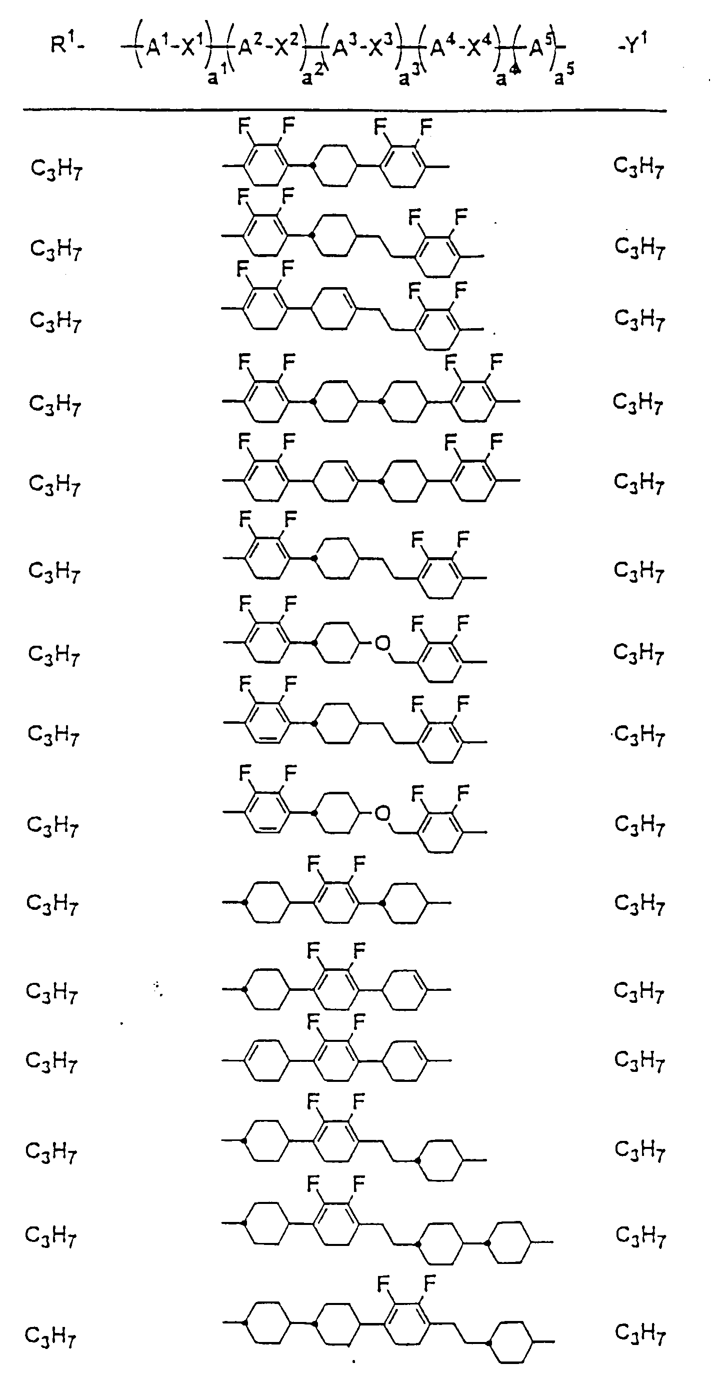

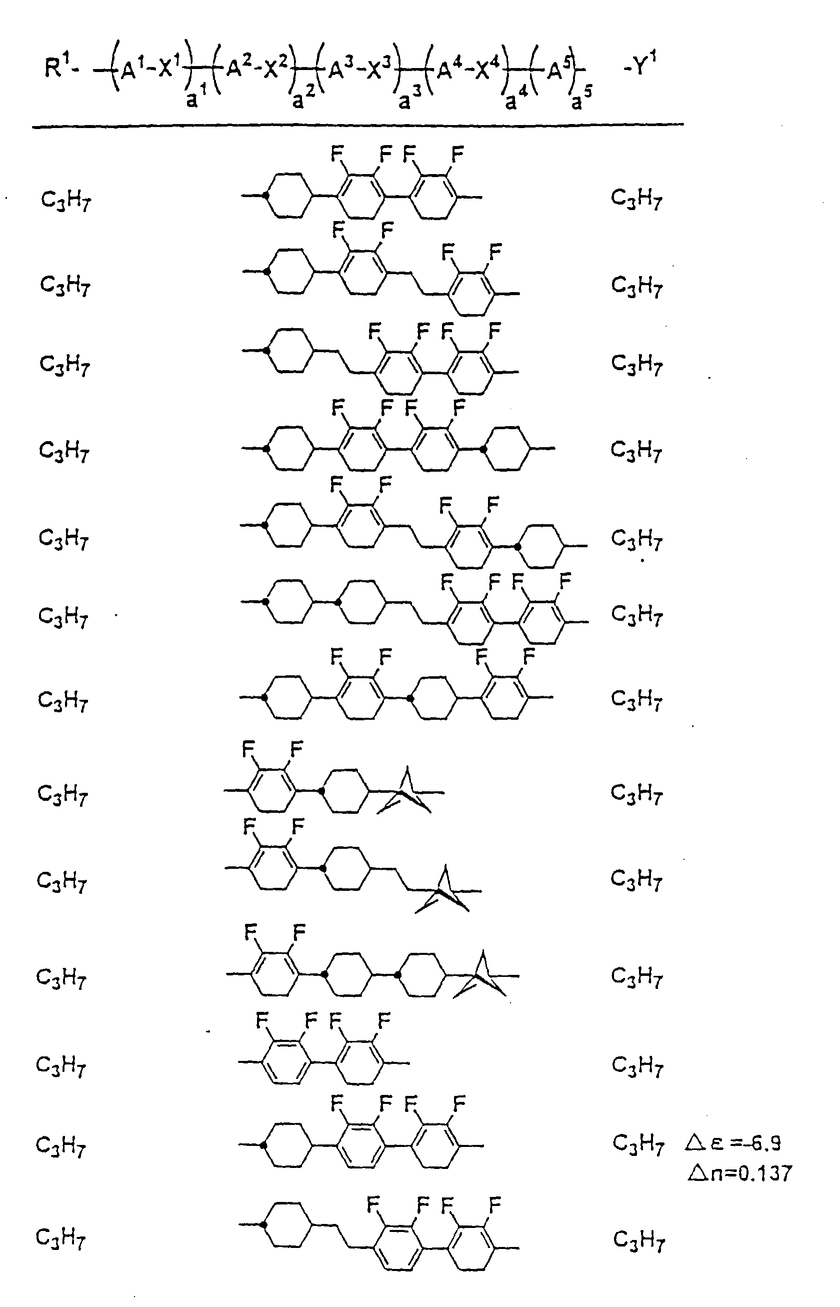

As has been described above in detail, the present invention can provide a liquid crystal compound which has a negative dielectric anisotropy value whose absolute value is large, a low viscosity, a controlled optical anisotropy value, a high specific resistance and a high voltage-holding ratio and which is stable against heat and irradiation with ultraviolet rays as well as a liquid crystal composition containing the compound and a liquid crystal display device produced using the composition.

## Claims

1. A liquid crystal compound having a 2,3-difluorocyclohexa-1, 3-diene-1,4-diyl group in the general structure of the compound.

2. The liquid crystal compound of claim 1 wherein it is represented by the following general formula (1):
R¹-(A¹-X¹)ₐ₁-(A²-X²)ₐ₂-(A³-X³)ₐ₃-(A⁴-X⁴)ₐ₄-(A⁵)ₐ₅-Y¹ (1)
wherein R¹ and Y¹ each independently represents an alkyl group having 1 to 20 carbon atoms, a hydrogen atom, a halogen atom, a cyano group, a cyanate residue, an isocyano group or an isothiocyanate residue, provided that at least one of the methylene groups present in the alkyl group, which are not adjacent to one another, may be substituted with an oxygen, sulfur or nitrogen atom, -C ≡C-, a silylene group wherein at least one hydrogen atom may be substituted with an alkyl group having 1 to 10 carbon atoms, or a vinylene group, or that at least one hydrogen atom of the alkyl group may be substituted with a fluorine or chlorine atom or that these alkyl groups may be optically active ones; X¹, X², X³ and X⁴ each independently represents a single bond, -(CH₂)₂-, -CH=CH-, -C≡C-, -COO-, -OCO-, -CH₂O-, -OCH₂-, -(CH₂)₄-, -(CH₂)₃O-, -O(CH₂)₃-, -CH=CHCH₂CH₂-, -CH₂CH=CHCH₂-, -CH₂CH₂CH=CH-, -CH=CHCH=CH-, -CF₂O-, -OCF₂-, -CH=CHCH₂O-, -OCH₂CH=CH-, -CF=CF-, -CH₂CF₂-, -CF₂CH₂-, -(CF₂)₂-, -(CF₂)₄-, -(CH₂)₂COO-, -OCO(CH₂)₂-, -CH=CHCOO-, -OCOCH=CH-, -CH=CHC≡C- or -C≡CCH=CH-; the rings A¹, A², A³, A⁴ and A⁵ each independently represents a 2,3-difluorocyclohexa -1,3-diene-1,4-diyl, bicyclo[1.1.1]pentane-1,3-diyl, trans-cyclohexane-1,4-diyl, cyclohexa-1-ene-1,4-diyl or 1,4-phenylene group, provided that the carbon atoms constituting these rings may be substituted with a nitrogen, oxygen or sulfur atom, that each hydrogen atom present on the rings may be substituted with a halogen atom or a cyano group and that at least one of these rings A¹, A², A³, A⁴ and A⁵ is 2,3-difluorocyclohexa-1,3-diene-1,4-diyl; a¹, a², a³, a⁴ and a⁵ each independently represents 0 or 1, provided that they satisfy the relation: a¹+a²+a³+a⁴+a⁵≧1; and each atom constituting the compound may be substituted with its isotope.

3. A liquid crystal composition comprising at least two components including at least one liquid crystal compound as set forth in claim 1 or 2.

4. A liquid crystal composition comprising at least one liquid crystal compound as set forth in claim 1 or 2 as a first component and at least one compound selected from the group consisting of those represented by the following general formulas (2), (3) and (4) as a second component: 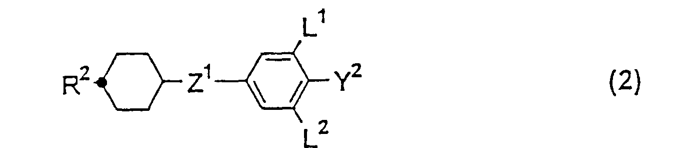 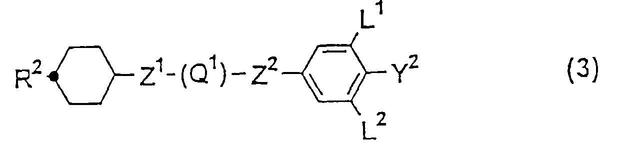 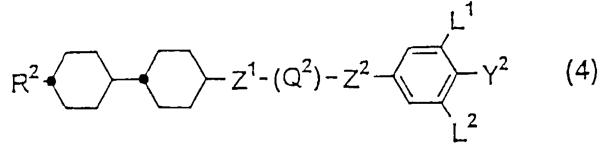 wherein R² represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y² represents a fluorine or chlorine atom, -OCF₃, -OCF₂H, -CF₃, -CF₂H, -CFH₂, -OCF₂CF₂H or -OCF₂CFHCF₃; L¹ and L² each independently represents a hydrogen or fluorine atom; Z¹ and Z² each independently represents -(CH₂)₂-, -(CH₂)₄-, -COO-, -CF₂O-, -OCF₂-, -CH=CH- or a single bond; the ring Q¹ represents a trans-cyclohexane-1,4-diyl or 1,3-dioxane-2,5-diyl group, or a 1,4-phenylene group whose hydrogen atom may be substituted with a fluorine atom; the ring Q² represents a trans-cyclohexane-1,4-diyl group, or a 1,4-phenylene group whose hydrogen atom may be substituted with a fluorine atom; and each atom constituting the compound may be substituted with its isotope.

5. A liquid crystal composition comprising at least one liquid crystal compound as set forth in claim 1 or 2 as a first component and at least one compound selected from the group consisting of those represented by the following general formulas (5) and (6) as a second component: 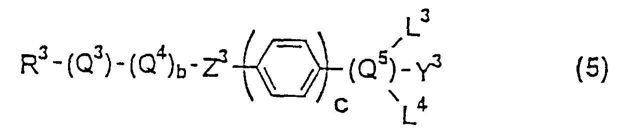 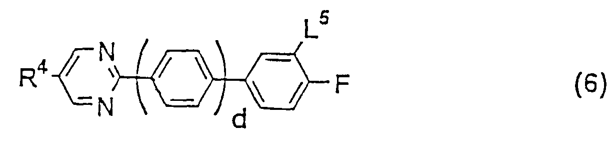 wherein R³ and R⁴ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; Y³ represents a cyano group or -C≡C-CN; the ring Q³ represents a trans-cyclohexane-1,4-diyl, 1,4-phenylene, 1,3-dioxane-2,5-diyl or pyrimidine-2,5-diyl group; the ring Q⁴ represents a trans-cyclohexane-1,4-diyl or pyrimidine-2,5-diyl group, or a 1,4-phenylene group whose hydrogen atom may be substituted with a fluorine atom; the ring Q⁵ represents a trans-cyclohexane-1,4-diyl or 1,4-phenylene group; Z³ represents -(CH₂)₂-, -COO- or a single bond; L³, L⁴ and L⁵ each independently represents a hydrogen or fluorine atom; b, c and d each independently represents 0 or 1; and each atom constituting the compound may be substituted with its isotope.

6. A liquid crystal composition comprising at least one liquid crystal compound as set forth in claim 1 or 2 as a first component; at least one compound selected from the group consisting of those represented by the foregoing general formulas (2), (3) and (4) as a second component; and at least one compound selected from the group consisting of those represented by the following general formulas (7), (8) and (9) as a third component:
R⁵-(Q⁶)-Z⁴-(Q⁷)-Z⁵-R⁶ (7)
R⁵-(Q⁶)-Z⁴-(Q⁷)-Z⁵-(Q⁸)-R⁶ (8) 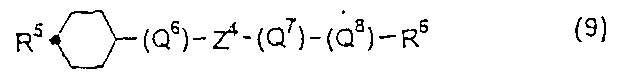
wherein R⁵ and R⁶ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁶, Q⁷ and Q⁸ each independently represents a trans-cyclohexane-1,4-diyl or pyrimidine-2,5-diyl group, or a 1,4-phenylene group whose hydrogen atom may be substituted with a fluorine ato m; Z⁴ and Z⁵ each independently represents -C≡C-, -COO-, -(CH₂)₂-, -CH=CH- or a single bond; and each atom constituting the compound may be substituted with its isotope.

7. A liquid crystal composition comprising at least one liquid crystal compound as set forth in claim 1 or 2 as a first component and at least one compound selected from the group consisting of those represented by the following general formulas (10), (11) and (12) as a second component: 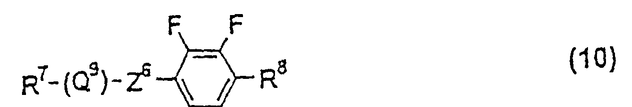 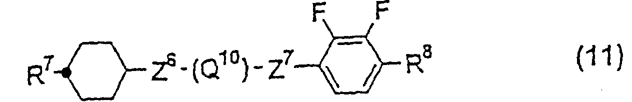 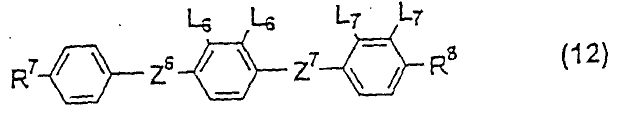 wherein R⁷ and R⁸ each independently represents an alkyl group having 1 to 10 carbon atoms, provided that at least one of the methylene groups present in the alkyl group, which are not adjacent to each other, may be substituted with an oxygen atom or -CH=CH- and that at least one of the hydrogen atoms present in the alkyl group may be substituted with a fluorine atom; the rings Q⁹ and Q¹⁰ each independently represents a trans-cyclohexane-1,4-diyl or 1,4-phenylene group; L⁶ and L⁷ each independently represents a hydrogen or fluorine atom, provided that L⁶ and L⁷ do not simultaneously represent a hydrogen atom; Z⁶ and Z⁷ each independently represents -(CH₂)₂-, -COO- or a single bond; and each atom constituting the compound may be substituted with its isotope.

8. A liquid crystal composition comprising at least one liquid crystal compound as set forth in claim 1 or 2 as a first component; at least one compound selected from the group consisting of those represented by the foregoing general formulas (7), (8) and (9) as a second component; and at least one compound selected from the group consisting of those represented by the foregoing general formulas (10), (11) and (1 2) as a third component.

9. A liquid crystal composition comprising at least one liquid crystal compound as set forth in claim 1 or 2 as a first component; at least one compound selected from the group consisting of those represented by the foregoing general formulas (5) and (6) as a second component; and at least one compound selected from the group consisting of those represented by the foregoing general formulas (7), (8) and (9) as a third component.

10. A liquid crystal composition comprising at least one liquid crystal compound as set forth in claim 1 or 2 as a first component; at least one compound selected from the group consisting of those represented by the foregoing general formulas (2), (3) and (4) as a second component; at least one compound selected from the group consisting of those represented by the foregoing general formulas (5) and (6) as a third component; and at least one compound selected from the group consisting of those represented by the foregoing general formulas (7), (8) and (9) as a fourth component.

11. A liquid crystal composition comprising at least one optically active compound in addition to one of the liquid crystal compositions as set forth in any one of claims 3 to 10.

12. A liquid crystal display device constituted by the use of one of the liquid crystal compositions as set forth in any one of claims 3 to 11.
